# EUROPÄISCHE PATENTSCHRIFT

(11) **EP 1 224 198 B1**
(45) Veröffentlichungstag und Bekanntmachung des Hinweises auf die Patenterteilung: **23.04.2003**
(21) Anmeldenummer: 00966152.1
(22) Anmeldetag: 10.10.2000
(51) Int. Cl.: C07H 19/06, C07H 19/16

(54) **NUCLEOSID-DERIVATE MIT PHOTOLABILEN SCHUTZGRUPPEN**
NUCLEOSIDE DERIVATIVES WITH PHOTOLABILE PROTECTING GROUPS
DERIVES DE NUCLEOSIDES A GROUPES PROTECTEURS PHOTOLABILES

(30) Priorität: 29.10.1999 DE 19952113
(43) Veröffentlichungstag der Anmeldung: 24.07.2002
(73) Patentinhaber: NIGU CHEMIE GMBH, D-84478 Waldkraiburg (DE)
(72) Erfinder: PFLEIDERER, Wolfgang, 78464 Konstanz (DE); BÜHLER, Sigrid, 78464 Konstanz (DE); GIEGRICH, Heiner, 84478 Waldkraiburg (DE)
(74) Vertreter: HOFFMANN - EITLE
(86) Internationale Anmeldenummer: EP0009958
(87) Internationale Veröffentlichungsnummer: WO01032671

(56) Entgegenhaltungen:
- US-A- 5 763 599
- H. GRIEGRICH ET AL.: "New photolabile protecting groups in nucleoside and nucleotide chemistry - synthesis, cleavage mechanisms and applications" NUCLEOSIDES & NUCLEOTIDES, Bd. 17, 1998, Seiten 1987-1996, XP002159918

## Beschreibung

### Technisches Gebiet

Gegenstand der vorliegenden Erfindung sind Nucleosid-Derivate mit photolabilen Schutzgruppen, Verfahren zu deren Herstellung und deren Verwendung.

### Stand der Technik

Photolabile Schutzgruppen für die Hydroxy- und Phosphatfunktionen in Nucleosiden bzw. Nucleotiden sind von besonderem Interesse, da sie bspw. für lichtgesteuerte Parallel-Synthesen von Oligonucleotiden auf einem festen Träger geeignet sind (vgl. S.P.A. Fodor et al., Science 1991, 251, S. 767 ff). Mit ihrer Hilfe können sogenannte DNA-Chips (d. h. Trägerplättchen, auf deren Oberfläche viele verschiedene Oligonucleotide angeordnet sind) hergestellt werden, die wiederum in der Molekularbiologie bspw. für Sequenz-Analysen oder Expressionsstudien benötigt werden.

Entsprechend dem Stand der Technik wurden bisher als photolabile Schutzgruppen in der Nucleosid- bzw. Nucleotidchemie vor allem die o-Nitrobenzyl-Gruppe und ihre Derivate eingesetzt (vgl. V.N.R. Pillai, Org. Photochem. 1987, 9, S. 225 ff bzw. J.W. Walker et al., J. Am. Chem. Soc. 1988,110, S 7170 ff). Des Weiteren wurden Schutzgruppen vom Pyrenylmethoxycarbonyltyp verwendet (vgl. WO 98/39 348). Als besonders nachteilig bei diesen Schutzgruppen hat sich die langsame und zum Teil nur unvollständige Entschützung der entsprechenden Nucleosid- bzw. Nucleotid-Derivate erwiesen. Außerdem entstehen bei der Abspaltung der o-Nitrobenzyl-Verbindungen z. T. unerwünschte Nebenprodukte in Form toxischer Nitrosophenylverbindungen.

Als weitere photolabile Schutzgruppen für die Nucleinsäurechemie wurden die 2-(2-Nitrophenyl)ethoxycarbonyl-Gruppe und die 2-(2-Nitrophenyl)ethylsulfonyl-Gruppe sowie deren Derivate eingeführt (vgl. WO 96/18 634 bzw. WO 97/44 345), die sich im Vergleich zu den oben genannten Nitrobenzyl- bzw. Pyrenylmethoxycarbonyl-Gruppen schneller und vollständiger abspalten lassen.

Als nachteilig bei diesen Schutzgruppen erwies sich die immer noch relativ langsame und unvollständige Entschützung der entsprechenden Nucleoside- bzw. Nucleotide.

### Darstellung der Erfindung

Der vorliegenden Erfindung lag daher die Aufgabe zugrunde, Nucleosid-Derivate mit photolabilen Schutzgruppen für die 5'-OH-Funktion im Zuckerteil zu entwickeln, welche die genannten Nachteile entsprechend dem Stand der Technik nicht aufweisen, sondern sich vergleichsweise schnell, quantitativ und ohne Bildung unerwünschter Nebenprodukte entschützen lassen.

Diese Aufgabe wurde erfindungsgemäß durch Nucleosid-Derivate der allgemeinen Formel (I) entsprechend Anspruch 1 gelöst. Es hat sich nämlich überraschenderweise gezeigt, dass sich die erfindungsgemäßen Schutzgruppen wesentlich schneller und vollständiger abspalten lassen als z. B. die o-Nitrobenzylgruppen. Außerdem konnten bei der Entschützung bisher keine Nebenprodukte in größerem Umfang festgestellt werden, was ebenfalls nicht vorhersehbar war.

Die erfindungsgemäßen Nucleosid-Derivate weisen folgende allgemeine Formel (I) auf: 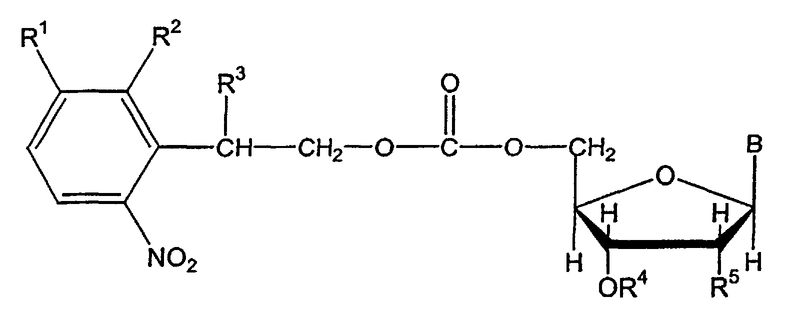 wobei die Reste R¹ und R² am Phenylring folgende Bedeutung haben können:
R¹ = H, F, Cl, Br, I, NO₂
R² = H, CN
und R¹ und R² nicht gleichzeitig H sind.
Der Rest R³, der am C₂-Atom der o-Nitrophenylethyl-Gruppierung sitzt, kann entweder H, ein Alkylrest mit 1 bis 4 C-Atomen oder ein Phenylrest sein. Der Alkylrest kann hierbei linear oder verzweigt sein.

Der Nucleosidteil der erfindungsgemäßen Verbindungen besteht aus den üblichen D-Ribofuranose- bzw. 2'-Desoxyribofuranose-Einheiten sowie den Pyrimidin- (B = Cytosin, Thymin, Uracil) bzw. Purinbasen (B = Adenin, Guanin). Als Basen können auch 2,6-Diaminopurin-9-yl-, Hypoxanthin-9-yl-, 5-Methylcytosin-1-yl-, 5-Amino-4-imidazolcarbonsäureamid-1-yl- oder 5-Amino-4-imidazolcarbonsäureamid-3-yl-Reste eingesetzt werden.

Die OH-Gruppe (n) im Ribofuranosid- bzw. 2'-Desoxyribofuranose-Teil können je nach Bedarf frei oder geschützt sein. Zum Schutz der 3'-Stellung (Position R⁴) haben sich hierbei die bekannten Phosphitamid-Gruppen bewährt, wie z. B. 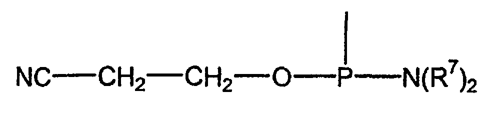 oder 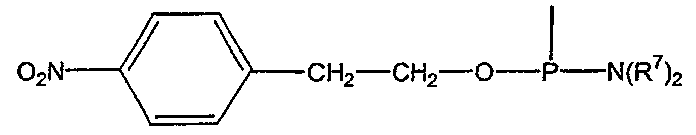 wobei die R⁷-Gruppen gleich oder verschieden sein können und lineare oder verzweigte Alkylreste mit 1 bis 4 C-Atomen bedeuten. Vorzugsweise sind sie Ethyl- oder Isopropylreste.

In der 2'-Stellung des Ribofuranosid-Teiles (Position R⁵) kann neben dem Wasserstoff- oder Halogenatom (insbesondere F, Cl, Br) eine freie oder geschützte OH-Gruppe vorliegen, wobei eine beliebige in der Nucleotidchemie übliche Schutzgruppe (R⁶) verwendet werden kann. Insbesondere kann auf die üblichen Alkyl-, Alkenyl-, Acetal- oder Silylether-Schutzgruppen für Sauerstoffatome (X = O) zurückgegriffen werden. R⁵ kann auch eine S-Alkylgruppe darstellen (X = S, R⁶ = Alkyl). Bevorzugte Beispiele für O-Alkyl-Schutzgruppen sind O-Methyl- oder O-Ethylreste, für O-Alkenyl-Schutzgruppen O-Allylreste, für O-Acetal-Schutzgruppen O-Tetrahydropyranyl- bzw. O-Methoxytetrahydropyranyl-Reste sowie für O-Silylether-Schutzgruppen O-t-Butyldimethylsilyl-Reste.

Gemäß einer bevorzugten Ausführungsform können die Pyrimidin- bzw. Purinbasen mit primären Aminofunktionen (z. B. Adenin, Cytosin und Guanin) noch permanente Schutzgruppen vorzugsweise auf Carbonylbasis aufweisen. Bevorzugt sind hierbei vor allem Phenoxyacetyl- oder Dimethylformamidino-Reste, die für alle drei genannten Basen in Frage kommen. Daneben gibt es noch spezielle Schutzgruppen, die nur bei bestimmten Basen eingeführt werden. Dies sind bspw. im Falle von Adenin Benzoyl- oder p-Nitrophenylethoxycarbonyl (p-NPEOC)-Reste. Für Guanin können neben den p-NPEOC-Resten auch Isobutyroyl- oder p-Nitrophenylethyl- (p-NPE)-Schutzgruppen (für die O-6-Funktion) eingeführt werden. Schließlich eignen sich für Cytosin neben den p-NPEOC-Resten auch noch Benzoyl- oder Isobutyroyl-Schutzgruppen.

Die Herstellung der erfindungsgemäßen Nucleosid-Derivate erfolgt in mindestens zwei Stufen. In der ersten Stufe a) wird ein Alkohol der allgemeinen Formel (II) 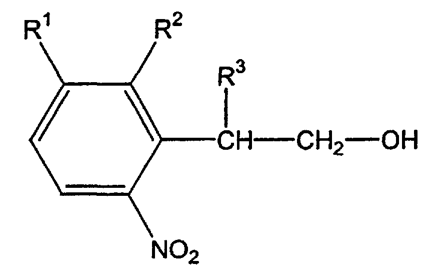 in der R¹, R², R³ die oben angegebene Bedeutung besitzen, mit einem Phosgen-Derivat vorzugsweise in einem unpolaren organischen Lösemittel bei Temperaturen zwischen - 20 und + 25 °C zur Umsetzung gebracht. Als Phosgen-Derivat kann neben Phosgen auch Diphosgen (Chlorameisensäuretrichlormethylester) oder Triphosgen (Bistrichlormethylcarbonat) verwendet werden.

Die Alkoholkomponente ist in den meisten Fällen bekannt oder kann in analoger Weise nach bekannten Verfahren hergestellt werden. Als unpolares organisches Lösemittel wird in Stufe a) vorzugsweise Toluol oder THF verwendet. Die Reaktionskomponenten können zwar in annährend stöchiometrischem Verhältnis eingesetzt werden, doch wird das Phosgen-Derivat vorzugsweise in deutlichem Überschuss, bezogen auf die Alkoholkomponente, eingesetzt. Auch die Konzentration der Alkoholkomponente kann in weiten Grenzen variiert werden, doch hat es sich als besonders vorteilhaft erwiesen, diese Konzentration auf 0,1 bis 10,0 mmol pro 10 ml Solvens einzustellen.

Bei dieser Reaktion (Reaktionsdauer ca. 1 bis 2 Std.) entstehen in guter Reinheit und hoher Ausbeute (> 95 %) die entsprechenden Chlorkohlensäureester der allgemeinen Formel (IV)

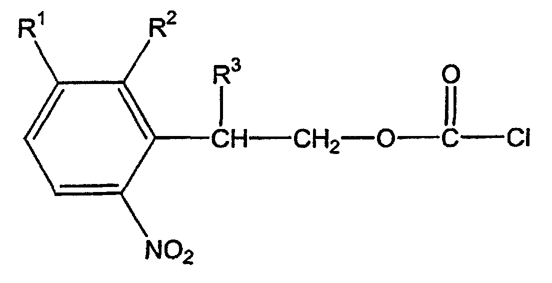

Die Aufarbeitung der entsprechenden Produkte erfolgt vorzugsweise, indem man zunächst das überschüssige Phosgen-Derivat sowie das Lösemittel im Vakuum abdestilliert. Der Chorkohlensäureester (IV) kann dann ohne weitere Aufarbeitung in Stufe b) mit den Nucleosiden der allgemeinen Formel (III) 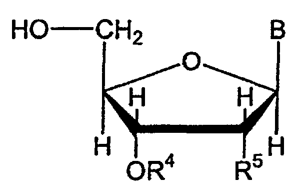 umgesetzt werden, wobei R⁴, R⁵ und B die oben angegebene Bedeutung besitzen.

Die Umsetzung erfolgt vorzugsweise in einem Lösemittelgemisch bestehend aus Dichlormethan und einem polaren organischen Lösemittel ggf. in Gegenwart einer Base bei Temperaturen zwischen - 60 und + 25 °C. Als polares organisches Lösemittel wird hierbei bevorzugt DMF oder Pyridin eingesetzt, wobei im Falle von Pyridin keine zusätzliche Base erforderlich ist. Wird jedoch mit Dichlormethan/DMF-Lösemittelgemischen gearbeitet, empfiehlt sich die Zugabe einer Base wie z. B. Pyridin, Triethylamin oder Ethyldiisopropylamin, um die bei der Reaktion freigesetzten Protonen abzufangen. Das Mischungsverhältnis Dichlormethan zu Pyridin bzw. DMF ist ebenfalls unkritisch, doch werden vorzugsweise 1 bis 3 Vol.-Teile Dichlormethan pro Vol.-Teil Pyridin bzw. DMF eingesetzt.

Gemäß einer bevorzugten Ausführungsform wird das entsprechende Nucleosid (III), welches in Pyridin oder DMF/Base gelöst wurde, vorgelegt und eine Lösung des Chlorkohlensäureesters in Dichlormethan bei der jeweiligen Reaktionstemperatur zugetropft. Das Molverhältnis von Nucleosid zu Chlorkohlensäureester kann hierbei entsprechend der Stöchiometrie auf ca. 1 : 1 eingestellt werden. Vorzugsweise wird jedoch der Chlorkohlensäureester im Überschuss eingesetzt, und zwar in einer solchen Menge, dass das Molverhältnis Nucleosid zu Chlorkohlensäureester 1 : 1 bis 1 : 2 beträgt. Schließlich kann auch die Konzentration des Nucleosids im Lösemittelgemisch in weiten Grenzen variiert werden, doch wird es bevorzugt auf 0,1 bis 3,0 mmol pro 10 ml Solvens eingestellt.

Nach erfolgter Umsetzung (Reaktionszeit ca. 5 bis 6 Std.) können die erfindungsgemäßen Nucleosid-Derivate nach bekannten Methoden isoliert bzw. gereinigt werden, wie z. B. Verdünnen mit Dichlormethan, Auswaschen aller Salze mit Wasser, Trocknen der organischen Phase, Einengen der Lösung bzw. Kristallisation und anschließende Kieselgel-Chromatographie. Auf diese Weise können die entsprechenden Nucleosid-Derivate mit hoher Reinheit und in guten Ausbeuten (ca. 70 bis 80 %) erhalten werden.

Gemäß einer bevorzugten Ausführungsform kann man im Anschluss an die Reaktionsstufe b) in die 3'-Stellung der Nucleosid-Derivate mit R⁴ = H die Phosphitamid-Gruppe 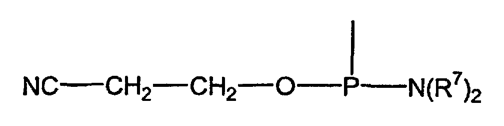 oder 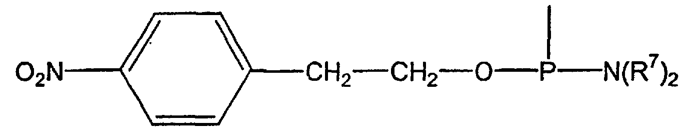 nach bekannten Methoden einführen. Üblicherweise erfolgt diese Umsetzung mit den entsprechenden Phosphinen in Gegenwart von 1H-Tetrazol als Aktivator in einem Lösemittelgemisch bestehend aus Dichlormethan und Acetonitril bei Temperaturen zwischen 0 und 25 °C. Vorzugsweise wird das Phosphin in 1,5- bis 3-fachem molarem Überschuss eingesetzt, während das Molverhältnis Phosphin zu 1H-Tetrazol auf 2 bis 4 : ca. 1,0 eingestellt wird. Das Mengenverhältnis von Dichlormethan zu Acetonitril ist relativ unkritisch und beträgt vorzugsweise 1 : 1 bis 4 : 1. Nach erfolgter Umsetzung kann das entsprechende Nucleosid wie in Stufe b) beschrieben aufgearbeitet werden.

Wie Bestrahlungsversuche mit polychromatischem Licht mit Wellenlängen > 289 nm belegen, lassen sich die erfindungsgemäßen Nucleoside sehr rasch (t _{0,5} = 20 bis 50 Sek.) und weitgehend entschützen (Ausbeuten bis zu 97 %), so dass die besonderen Anforderungen an die Photolabilität der Schutzgruppen in hervorragender Weise erfüllt werden.

Aufgrund dieser besonderen Eigenschaften eignen sich die erfindungsgemäßen Nucleoside sehr gut für die Herstellung von Oligonucleotiden durch lichtgesteuerte Schutzgruppenabspaltung insbesondere auf festen Trägerplatten.

Die nachfolgenden Beispiele sollen die Erfindung näher erläutern.

### Beispiele

### A) Synthese der Alkohol-Vorstufen

### Beispiel A.1

### 3-Acetylamino-1-ethylbenzol (1) [I]

### [I] H. Wieland und L. Horner, Liebigs Ann., 536, 89 (1938)

Zu 100 ml Acetanhydrid werden unter Eisbadkühlung 27 g (25 ml, 0,22 mol) 3-Ethylanilin gegeben, nach 10 min im Eisbad rührt man noch 20 min bei RT und rotiert dann so weit wie möglich ein. Das Rohprodukt (36,6 g) wird über Destillation im Hochvakuum gereinigt. Man erhält 32,1 g (0,2 mol, 89 %) (1) als leicht gelblichen Feststoff mit einem Siedepunkt von 110-114 °C (0,05 mbar).

Physikalische Daten von (1):
Schmp.. 30-32 °C (Lit [I] : 33-34 °C)
DC (Kieselgel, PE/EE 1:1): R_{f} = 0,42
¹H-NMR (250 MHz, CDCl₃): 7,60 (s(br), 1H, NH), 7,34-7,16 (m, 3H, arom. H), 6,92 (d, 1H, arom. H), 2,59 (q, 2H, CH₂), 2,14 (s, 3H, COCH₃), 1,18 (t, 3H, CH₃)
Uv-Spektrum (MeOH), λ ₘₐₓ [nm] (log ε) : 206 (3,41), 242 (3,10), [280 (1,76)]

### Beispiel A.2

5-Amino-1-ethyl-2-nitrobenzol [I] (2),
3-Amino-1-ethyl-2,4-dinitro-benzol (3),
3-Amino-1-ethyl-2,6-dinitrobenzol (4) und
5-Amino-1-ethyl-2,4-dinitrobenzol (5)

### [I] H. Wieland und L. Horner, Liebigs Ann., 536, 89 (1938)

Für diese Reaktion ist ein KPG - Rührer empfehlenswert! Zu 70 ml konz. Schwefelsäure, auf -20 °C gekühlt, gibt man 15 g (92 mmol) 3-Acetylamino-1-ethylbenzol (1) so zu, dass die Temperatur unterhalb von 0 °C bleibt. Daraufhin werden 9,5 g (6,2 ml, 0,15 mmol) rauchende Salpetersäure innerhalb von 30 min so zugetropft, dass die Temperatur nicht über -3 °C steigt. Nach 1h Rühren im Eisbad gießt man die Reaktionsmischung auf Eis, neutralisiert mit festem Natriumcarbonat und extrahiert 1 x mit 400 ml und 2 x mit je 200 ml Et₂O. Nach Trocknung über Na₂SO₄ und Einrotieren wird das erhaltene braune Öl mit100 ml konz. HCl 2 h bis zum Sieden erhitzt. Nach dem Abkühlen wird der ausgefallene Niederschlag abgesaugt, in 1 n NaOH aufgeschlämmt und 3 x mit je 100 ml Et₂O extrahiert. Die organische Phase trocknet man über Na₂SO₄ und rotiert ein. Das Rohprodukt wird auf Kieselgel aufgezogen und über Flash-Chromatographie (127 g Kieselgel, 6 x 13 cm, LM:PE/EE, kond. 8:1, Gradient: von 8:1 bis 4:1) vorgereinigt. Man erhält 110 mg (0,5 mmol, 0,6 %) (3), 790 mg (4 mmol, 4 %) (4) und 7 g eines Gemisches von (1) und (5). Dieses Gemisch kann durch Aufkochen in 15 ml HCl/75 ml H₂O getrennt werden. Das gewünschte Produkt bleibt als Hydrochlorid in Lösung, während das Dinitroderivat als Feststoff (oder Öl) zurückbleibt. Man dekantiert die Flüssigkeit ab, neutralisiert mit Natriumcarbonat, extrahiert 3 x mit je 50 ml Et₂O, trocknet über Na₂SO₄ und rotiert ein. Man erhält 4,3 g (26 mmol, 28 %) (2) als gelben Feststoff. Aufnehmen des Rückstandes in Et₂O, Trocknung über Na₂SO₄ und Einrotieren liefert 2,34 g (11 mmol, 12 %) (5) als braunen Feststoff.

Physikalische Daten von (2):
Schmp.: 84-85 °C (Lit [I]: 80-81 °C)
DC (Kieselgel, PE/EE 7:3): R_{f} = 0,27
¹H-NMR (250 MHz, CDCl₃): 7,96 (m, 1H, H(6)), 6,47 (m, 2H, H(3), H(5)), 4,23 (s(br), 2H, NH₂), 2,93 (q, 2H, CH₂), 1,24 (t, 3H, CH₃)
¹³C-NMR (600 MHz, CDCl₃): 151,30 (C(5)), 143, 25 (C(1)), 139,42 (C(2)), 128,35 (C(3)), 115,35 (C(6)), 111,72 (C(4)), 27,48 (CH₂), 14,67 (CH₃)
UV-Spektrum (MeOH), λ ₘₐₓ [nm] (log ε): 203 (4,21), 231 (3,82), [245 (3,69)], [372 (4,09)]
MS (EI 70 eV, m/z (%)): M⁺ 166 (55), 149 (100), 121 (42), 93 (26), 65 (35), 52 (23), 39 (32)

Physikalische Daten von (3) :
Schmp.: 85-89 °C
DC (Kieselgel, PE/EE 7:3) : R_{f} = 0,77
¹H-NMR (600 MHz, CDCl₃) : 8,24 (d, 1H, H(5)), 7,02 (s(br), 2H, NH₂), 6,66 (d, 1H, H(6)), 2,70 (q, 2H, CH₂), 1,26 (t, 3H, CH₃)
¹³C-NMR (600 MHz, CDCl₃) : 147,46 (C(1)), 139,02 (C(2)), 138,80 (C(3)), 132,23 (C(4)), 129,40 (C(5)), 116,86 (C(6)), 26,57 (CH₂), 14,42 (CH₃)
UV-Spektrum (MeOH), λ ₘₐₓ [nm] (log ε) : 202 (4,08), 225 (4,27), 270 (3,78), 399 (3,72)

Physikalische Daten von (4):
Schmp.: 103-106 °C
DC (Kieselgel, PE/EE 7:3): R_{f} = 0,43
¹H-NMR (600 MHz, CDCl₃) : 7,95 (d, 1H, H(5)), 6, 70 (d, 1H, H(4)), 4,96 (s(br), 2H, NH₂), 2,92 (q, 2H, CH₂) , 1,31 (t, 3H, CH₃)
¹³C-NMR (600 MHz, CDCl₃) : 143, 94 (C(3)), 139,83 (C(6)), 137,48 (C(2)), 136,16 (C(1)), 129,32 (C(5)), 114,67 (C(4)), 22,29 (CH₂), 14,62 (CH₃)
UV-Spektrum (MeOH), λ ₘₐₓ [nm] (log ε): 202 (4,19), 225 (3,93),346 (3,94)

Physikalische Daten von (5) :
Schmp.: 96-101 °C
DC (Kieselgel, PE/EE 7:3) : R_{f} = 0,27
¹H-NMR (250 MHz, CDCl₃) : 8,98 (s, 1H), 6,69 (s, 1H), 6,51 (s(br), 2H, NH₂), 2,98 (q, 2H, CH₂), 1,26 (t, 3H, CH₃)
¹³C-NMR (600 MHz, CDCl₃) : 147,97 (C(1)), 146,95 (C(5)), 138,16 (C(2)), 129,03 (C(4)), 125,70 (C(3)), 119,59 (C(6)), 27,13 (CH₂), 14,19 (CH₃)
UV-spektrum (MeOH), λ ₘₐₓ [nm] (log ε) : 202 (4,15), [228 (4,01)], 265 (3,99), 342 (4,02), [361 (4,00)]
MS (EI 70 eV, m/z (%)): M⁺ 211 (56), 194 (100), 148 (26), 118 (30), 91 (30), 65 (24), 52 (38), 39 (31)

### Beispiel A.3

### 5-Chlor-1-ethyl-2-nitrobenzol (6)

1,66 g (10 mmol) 5-Amino-1-ethyl-2-nitrobenzol (2) werden in einer 60 °C warmen Mischung aus 5 ml konz. Salzsäure und 25 ml H₂O gelöst und im Eisbad schnell abgekühlt. Bei einer Temperatur unterhalb von 5 °C diazotiert man mit 760 mg (11 mmol) Natriumnitrit in 10 ml H₂O, gibt nach 10 min eine Spatelspitze Harnstoff hinzu und rührt weitere 5 min im Eisbad. Diese Reaktionsmischung wird in eine 80 °C warme Lösung von 1,5 g Kupfer(I)chlorid in 10 ml konz. Salzsäure und 5 ml H₂O gegeben, wobei eine starke Gasentwicklung auftritt. Die Lösung hält man noch 30 min bei dieser Temperatur. Nach dem Abkühlen wird die Reaktionslösung 3 x mit je 50 ml EE extrahiert und die vereinigten organischen Phasen noch je 1 x mit je 50 ml NaOH und H₂O gewaschen. Man trocknet über Na₂SO₄ und rotiert ein. Das Rohprodukt wird auf Kieselgel aufgezogen und durch Flash-Chromatographie (40 g Kieselgel, 3,5 x 13 cm, LM: PE) gereinigt. Man erhält 863 mg (4,6 mmol, 46 %) (6) als gelbes Öl.

Physikalische Daten von (6) :
DC (Kieselgel, PE/EE 4:1) : R_{f} = 0,77
¹H-NMR (250 MHz, CDCl₃): 7,85 (d, 1H, H(3)), 7,34 (d, 1H, H(6)), 7,29 (dd, 1H, H(4)), 2,90 (q, 2H, CH₂), 1,27 (t, 3H, CH₃)
UV-Spektrum (MeOH), λ ₘₐₓ [nm] (log ε): 206 (4,09), [216 (3,91)], 263 (3,79), [334 (2,84)]
C₈H₈ClNO₂ (185,61 g/mol):
ber.: C 51,77, H 4,34, N 7,55; gef.: C 51,65, H 4,34, N 7,63

### Beispiel A.4

### 5-Brom-1-ethyl-2-nitrobenzol (7)

In eine auf 60 °C erwärmte Mischung aus 12,5 ml 48 %-iger Bromwasserstoffsäure und 7,5 ml H₂O werden 1,66 g (10 mmol) 5-Amino-1-ethyl-2-nitrobenzol (2) eingetragen und im Eisbad schnell abgekühlt. Bei einer Temperatur unterhalb von 5 °C diazotiert man mit 760 mg (11 mmol) Natriumnitrit in 10 ml H₂O, gibt nach 10 min eine Spatelspitze Harnstoff hinzu und rührt weitere 5 min im Eisbad. Diese Reaktionsmischung wird in eine Suspension von 1,5 g (6 mmol) Kupfersulfatpentahydrat und 600 mg (9,4 mmol) Kupferpulver in 12,5 ml 48 %-iger Bromwasserstoffsäure und 7,5 ml H₂O gegeben und 30 min auf 80 °C erwärmt. Nach dem Abkühlen wird die Reaktionslösung 3 x mit je 50 ml EE extrahiert und die vereinigten organischen Phasen je 1 x mit je 50 ml In NaOH und H₂O gewaschen. Man trocknet über Na₂SO₄ und rotiert ein. Das Rohprodukt wird auf Kieselgel aufgezogen und durch Flash-Chromatographie (40 g Kieselgel, 3,5 x 13 cm, LM: PE/EE, kond. PE, Gradient: 150 ml PE, 600 ml 150:1, 150 ml 150:2) gereinigt. Man erhält 1,55 g (6,7 mmol, 67 %) (7) als gelbes Öl.

Physikalische Daten von (7):
DC (Kieselgel, PE/EE 4:1): R_{f} = 0,76
¹H-NMR (250 MHz, CDCl₃) : 7,76 (d, 1H, H(3)), 7,50 (d, 1H, H(6)), 7,45 (dd, 1H, H(4)), 2,89 (q, 2H, CH₂), 1,27 (t, 3H, CH₃)
UV-Spektrum (MeOH), λ ₘₐₓ [nm] (log ε) : 203 (4,19), [217 (3,98)], 267 (3,91), [320 (3,21)]
C₈H₈BrNO₂ (230,07 g/mol) :
ber.: C 41,77, H 3,50, N 6,09; gef.: C 41,71, H 3,59, N 6,08

### Beispiel A.5

### 1-Ethyl-5-iod-2-nitrobenzol (8)

In einer auf 50 °C erwärmten Mischung aus 3 ml konz. Schwefelsäure und 20 ml H₂O werden 1,66 g (10 mmol) 5-Amino-1-ethyl-2-nitrobenzol (2) gelöst und im Eisbad schnell abgekühlt. Bei einer Temperatur unterhalb von 5 °C diazotiert man mit 760 mg (11 mmol) Natriumnitrit, gibt nach 10 Minuten eine Spatelspitze Harnstoff hinzu und rührt weitere 5 Minuten im Eisbad. Diese Reaktionsmischung wird in eine Lösung von 2,5 g Kaliumjodid (15 mmol) in 10 ml H₂O gegeben und 1 h bei RT gerührt. Die Reaktionsmischung wird 3 x mit je 50 ml EE extrahiert und die vereinigten organischen Phasen je 1 x mit je 50 ml ln NaOH und H₂O gewaschen. Man trocknet über Na₂SO₄ und rotiert ein. Das Rohprodukt wird auf Kieselgel aufgezogen und durch Flash-Chromatographie (40 g Kieselgel, 3,5 x 13 cm, LM: PE) gereinigt. Man erhält 1,57 g (5,7 mmol, 57 %) (8) als rotes Öl.

Physikalische Daten von (8) :
DC (Kieselgel, PE/EE 4:1): R_{f} = 0,78
¹H-NMR (250 MHz, CDCl₃) : 7,72 (d, 1H, H(6)), 7,66 (dd, 1H, H(4)), 7,58 (d, 1H, H(3)), 2,85 (q, 2H, CH₂), 1,25 (t, 3H, CH₃)
UV-Spektrum (MeOH), λ ₘₐₓ [nm] (log ε) : 203 (4,22), [220 (3,88)], 281 (3,87), [328 (3,22)]
C₈H₈INO₂ (277,06 g/mol) :
ber.: C 34,68, H 2,91, N 5,06; gef.: C 34,84, H 2,92, N 4,90

### Beispiel A.6

### 4-Amino-2-nitro-ethylbenzol (30) [II]

### [II] A. Kövendi und M. Kircz, Chem. Ber., 97, 1896 (1964)

In einem 1 l Dreihalskolben mit KPG-Rührer, Innenthermometer und Tropftrichter kühlt man 380 ml konz. Schwefelsäure (d 1,84 g/ml) auf 8 °C und tropft dazu langsam 60,59 g (62 ml, 0,5 mol) 4-Ethylanilin, so dass die Temperatur konstant bleibt. Nach Ende des Zutropfens kühlt man auf -5 °C ab. Währenddessen werden unter Eiskühlung zu 23 ml 100 %-iger Salpetersäure (1,52 g/ml) 54 ml konz. Schwefelsäure (1,84 g/ml) zugetropft. Dieses Gemisch wird dann langsam zu der 4-Ethylanilin-Lösung zugetropft, so dass die Temperatur zwischen -5 und 0 °C gehalten wird. Es wird noch 45 min bei dieser Temperatur gerührt. Danach gießt man auf Eis und saugt den ausgefallenen Niederschlag ab. Der Niederschlag wird in 500 ml H₂O suspendiert, auf 50 °C erhitzt und solange NH₃ eingeleitet, bis ein pH von 8 erreicht ist. Damit die Temperatur zwischen 50 und 60 °C gehalten wird, ist Eisbadkühlung notwendig. Es scheidet sich ein dunkelbraunes Öl ab, das bei 10 °C kristallisiert. Der Niederschlag wird abgesaugt und im Exsikkator über CaCl₂ getrocknet. Man erhält 57,7 g (0,35 mol, 70 %) (30) als braunen Feststoff, der ungereinigt weiter verwendet wird.

Physikalische Daten von (30) :
Schmp.: 30-31 °C (Lit. [2] : 44-45 °C)
DC (Kieselgel, Tol/EE 3:1) : R_{f} = 0,34
¹H-NMR (250 MHz, CDCl₃): 7,10 (d, 1H, H(6)), 7,03 (d, 1H, H(3)), 6,80 (dd, 1H, H(5)), 5,54 (s, 2H, NH₂), 2,60 (q, 2H, CH₂), 1,10 (t, 3H, CH₃)

### Beispiel A.7

### 4-Acetamido-1-ethyl-2-nitrobenzol (31) [III]

### [III] O. L. Brady, J. N. E. Day und P. S. Allam, J. Chem. Soc., 978 (1928)

50 g (0,3 mol) 4-Amino-1-ethyl-2-nitrobenzol (30) werden in 250 ml eiskaltes Acetanhydrid gegeben. Man lässt 90 min bei RT rühren und gießt auf Eis. Der ausgefallene Niederschlag wird abgesaugt und im Exsikkator über KOH getrocknet. Man erhält 58,18 g (0,28 mmol, 93 %) (31) als fast farblosen Feststoff.

Physikalische Daten von (31) :
Schmp.. 112-113 °C (Lit. [III]: 111 °C)
DC (Kieselgel, PE/EE 1:1): R_{f} = 0,35
¹H-NMR (250 MHz, CDCl₃) : 8,01 (d, 1H, H(3)), 7,71 (dd, 1H, H(5)), 7,62 (s(br.). 1H, NH),
7,27 (d, 1H, H(6)), 2,84 (q, 2H, CH₂), 2,19 (s, 3H, C(O)CH₃), 1,23 (t, 3H, CH₃)
UV-Spektrum (MeOH), λ ₘₐₓ [nm] (log ε) : 202 (4,23), 241 (4,39), 333 (3,21)

### Beispiel A.8

4-Amino-1-ethyl-2,3-dinitrobenzol (32) und
4-Amino-1-ethyl-2,5-dinitrobenzol (33) [III]

### [III] O. L. Brady, J. N. E. Day und P. S. Allam, J. Chem. Soc., 978 (1928)

20,3 g (0,1 mol) 4-Acetamido-1-ethyl-2-nitrobenzol (31) werden zu einer Mischung von 80 ml 65 %-iger Salpetersäure und 80 ml konz. Schwefelsäure gegeben. Man lässt 3 Tage bei RT rühren und gießt auf Eis. Der ausgefallene Niederschlag wird abgesaugt, in CH₂Cl₂ aufgenommen, mit 100 ml H₂O versetzt und mit festem Na₂CO₃ neutralisiert. Die organische Phase wird über Na₂SO₄ getrocknet und einrotiert. Das erhaltene Rohprodukt (20,6 g, 81 mmol, 81 %) wird in 200 ml Ethanol suspendiert und mit 2 g festem Natriumhydroxid 40 min zum Sieden erhitzt. Nach dem Abkühlen rotiert man die Reaktionsmischung zusammen mit 28 g Kieselgel bis zur Trockene ein. Die Reinigung erfolgt über Flash-Chromatographie (140 g Kieselgel, 6 x 14 cm, LM:PE/EE, kond.: 7:1, Gradient: von 7:1 bis 2:1). Man erhält 1,75 g (8 mmol, 8 %) (33) als orangeroten Feststoff und 12,5 g (60 mmol, 60 %) (32) als orangen Feststoff.

Physikalische Daten von (32) :
Schmp. : 122-123 °C (Lit. [III]: 125 °C)
DC (Kieselgel, PE/EE 1:1): R_{f} = 0,50
¹H-NMR (250 MHz, CDCl₃) : 7,26 (d, 1H, arom. H), 6,90 (d, 1H, arom. H), 6,03 (s(br.), 2H, NH₂), 2,45 (q, 2H, CH₂), 1,18 (t, 3H, CH₃)
UV-Spektrum (MeOH), λ ₘₐₓ [nm] (log ε) : 202 (4,18), 227 (4,37), [265 (3,59)], 411 (3,75)

Physikalische Daten von (33) :
Schmp.: 124-125 °C (Lit. [III]: 121,6 °C)
DC (Kieselgel, PE/EE 1:1): R_{f} = 0,85
¹H-NMR (250 MHz, CDCl₃): 8,11 (s, 1H, arom. H), 7,27 (s, 1H, arom. H), 6,07 (s(br.), 2H, NH₂), 2,75 (q, 2H, CH₂), 1,24 (t, 3H, CH₃)
UV-Spektrum (MeOH), λₘₐₓ [nm] (log ε): 226 (4,39), [243 (4,16)], [266 (3,86)], 422 (3,71)

### Beispiel A.9

### 1-Ethyl-2,5-dinitrobenzol (34) [III, IV]

### [III] O. L. Brady, J. N. E. Day und P. S. Allam, J. Chem. SoC., 978 (1928)

### [IV] M. M. A. F. Holleman und J. Böeseken, Rec. Trav. Chim., 16, 425 (1897)

4 g (19 mmol) 4-Amino-1-ethyl-2,5-dinitrobenzol (33) werden in 50 ml konz. Schwefelsäure gelöst und unter starker Kühlung mit 50 ml H₂O versetzt. Man lässt 1,45 g (18 mmol) Natriumnitrit in 15 ml H₂O langsam zutropfen (Reaktionstemperatur unter 10 °C). Nach Ende der Zugabe wird die Reaktionsmischung noch 10 min im Eisbad gerührt und dann in 100 ml siedendes EtOH eingetragen. Nach 5 min am Rückfluss lässt man abkühlen, gießt auf Eis und extrahiert 1 x mit 200 ml und 3 x mit je 100 ml Et₂O. Die vereinigten organischen Phasen werden 1 x mit 200 ml ges. NaHCO₃-Lösung gewaschen und die wässrige Phase mit 50 ml Et₂O rückextrahiert. Nach Trocknung über Na₂SO₄ rotiert man ein und reinigt das Rohprodukt (3,55 g rotes Öl, auf 4 g Kieselgel aufgezogen) über Flash-Chromatographie (67g Kieselgel, 4 x 12 cm, LM:PE/EE 8:1). Man erhält 2,64 g (13 mmol, 71 %) (34) als gelborangen Feststoff.

Physikalische Daten von (34) :
Schmp.: 57-60 °C (Lit. [III]: 57,4-59,9 °C)
DC (Kieselgel, PE/EE 4:1) : R_{f} = 0,60
¹H-NMR (250 MHz, CDCl₃) : 8,24 (d, 1H, H(6)), 8,16 (dd, 1H, H(4)), 7,95 (d, 1H, H(3)), 2,95 (q, 2H, CH₂), 1,34 (t, 3H, CH₃)
UV-Spektrum (MeOH), λ ₘₐₓ [nm] (log ε) : 204 (4,26), 257 (4,02), [298 (3,37)], [332 (3,06)]

### B) Synthese der Alkohole

### Allgemeine Arbeitsvorschrift (AAV) 1 zur Umsetzung mit Paraformaldehyd

AAV 1: 5 mmol Edukt werden mit 5 mmol Paraformaldehyd in 10 ml DMSO (z. S., über Molsieb getrocknet) gelöst. Bei der Zugabe von 0,05-0,5 mmol Kalium-tert.-butylat in 3 ml tert.-Butanol tritt ein Farbumschlag von gelb nach violett auf. Nach 15 min Rühren bei RT und 2 h bei 80 °C lässt man die Lösung abkühlen, neutralisiert mit ungefähr 4 Tropfen konz. HCl, verdünnt mit 25 ml gesättigter NaCl-Lösung und extrahiert 3 x mit je 20 ml Essigester. Die vereinigten organischen Phasen werden über Na₂SO₄ getrocknet, abfiltriert und einrotiert. Das Rohprodukt wird durch Flash-Chromatographie gereinigt (45 g Kieselgel, 4 x 12 cm, LM:Tol/EE, kond.: Tol, Gradient: Tol, bis nicht umgesetztes Edukt abgetrennt ist, dann 15:1 oder 8:1).

AAV 1.2: Durchführung entsprechend AAV 1, nur mit DMF anstelle von DMSO als Lösemittel, und die Reaktionszeit wird auf 3 h bei 90 °C erhöht.

### Beispiel B.1

### 2-(5-Chlor-2-nitrophenyl)propanol (9)

Nach AAV 1: 895 mg (4,8 mmol) 5-Chlor-1-ethyl-2-nitrobenzol (6), 160 mg (5,3 mmol) Paraformaldehyd in 10 ml DMSO und 76 mg (0,7mmol) Kalium-tert.-butylat in 4 ml tert.-Butanol. Ausbeute: 870 mg (4,03 mmol, 84 %) (9) als gelbes Öl.

Physikalische Daten von (9) :
DC (Kieselgel, PE/EE 4:1) : R_{f} = 0,27
¹H-NMR (250 MHz, CDCl₃): 7,72 (d, 1H, H(3)), 7,45 (d, 1H, H(6)), 7,30 (dd, 1H, H(4)), 3,74 (m, 2H, α-CH₂), 3,54 (sextett, 1H, β-CH), 1,71 (s(br.), 1H, OH), 1,30(d, 3H, CH₃) UV-Spektrum (MeOH), λₘₐₓ [nm] (log ε): 206 (4,17), 216 (4,06), 262 (3,76), [328 (2,95)]
C₉H₁₀ClNO₃ (215, 64 g/mol) :
ber.: C 50,13, H 4,67, N 6,50; gef.: C 50,20, H 4,75, N 6,20

### Beispiel B.2

### 2- (5-Brom-2-nitrophenyl)propanol (10)

Nach AAV 1: 1,39 g (6 mmol) 5-Brom-1-ethyl-2-nitrobenzol (7), 210 mg (7 mmol) Paraformaldehyd in 10 ml DMSO und 100 mg (0,89 mmol) Kalium-tert.-butylat in 4 ml tert.-Butanol. Ausbeute: 1,32 g (5,01 mmol, 85 %) (10) als gelbes Öl.

Physikalische Daten von (10):
DC (Kieselgel, PE/EE 7:3): R_{f} = 0,57
¹H-NMR (250 MHz, CDCl₃) : 7,63 (m, 2H, H(3), H(6)), 7,47 (dd, 1H, H(4)), 3,74 (m, 2H,α-CH₂), 3,52 (sextett, 1H, β-CH), 1,68 (s (br.), 1H, OH), 1,30(d, 3H, CH₃)
UV-Spektrum (MeOH), λ ₘₐₓ [nm] (log ε): 202 (4,16), [218 (3,95)], 263 (3,76), [330 (2,96)]
C₉H₁₀BrNO₃ (260,09 g/mol):
ber.: C 41,56, H 3,88, N 5,39; gef.: C 41,74, H 3,95, N 5,27

### Beispiel B.3

### 2-(5-Iod-2-nitrophenyl)propanol (11)

Nach AAV 1: 1,45 g (5 mmol) 5-Iod-1-ethyl-2-nitrobenzol (8), 165 mg (5,5 mmol) Paraformaldehyd in 10 ml DMSO und 80 mg (0,7 mmol) Kalium-tert.-butylat in 4 ml tert.-Butanol.
Ausbeute: 1,36 g (4,4 mmol, 85 %) (11) als gelben Feststoff.

Physikalische Daten von (11) :
DC (Kieselgel, PE/EE 4:1): R_{f} = 0,29
¹H-NMR (250 MHz, CDCl₃) : 7,81 (d, 1H, H(6)), 7,69 (dd, 1H, H(4)), 7,46 (d, 1H, H(3)), 3,76 (m, 2H, α-CH₂), 3,48 (sextett, 1H, β-CH), 1,54 (s (br.), 1H, OH), 1,29 (d, 3H, CH₃)
UV-Spektrum (MeOH), λₘₐₓ [nm] (log ε): 204 (4,29), [222 (3,93)], 274 (3,8), [335 (3,18)]
C₉H₁₀INO₃ (307,08 g/mol):
ber.: C 35,17, H 3,28, N 4,56; gef.: C 35,18, H 3,27, N 4,06

### Beispiel B.4

### 2-(2,5-Dinitrophenyl)propanol (35)

Nach AAV1.2: 1,44 g (7 mmol) 1-Ethyl-2,5-dinitrobenzol (34), 662 mg (22 mmol) Paraformaldehyd in 10 ml DMF und 200 mg (1,8 mmol) Kalium-tert.-butylat in 4 ml tert.-Butanol.
Ausbeute: 628 mg (3,2 mmol, 46 %) Edukt (34) und 394 mg (1,74 mmol, 25 %) (35) als gelber Feststoff. Für die Analyse werden 100 mg (35) aus 15 ml H₂O/EtOH 4:1 umkristallisiert.

Physikalische Daten von (35):
Schmp.. 82-83 °C
DC (Kieselgel, PE/EE 7:3): R_{f} = 0,45
¹H-NMR (250 MHz, CDCl₃): 8,38 (d, 1H, H(6)), 8,19 (dd, 1H, H(4)), 7,84 (d, 1H, H(3)), 3,90-3,72 (m, 2H, 2 x α-CH), 3,52 (sextett, 1H, β-CH ), 1,55 (t, 1H, OH), 1,38 (d, 3H, CH₃)
UV-Spektrum (MeOH), λ ₘₐₓ [nm] (log ε): 205 (4,24), 256 (4,00), [289 (3,41)] , [334 (3,04)]
C₉H₁₀N₂O₅ (226,19 g/mol):
ber.: C 47,79, H 4,46, N 12,39; gef.: C 48,01, H 4,42, N 12,33

### C) Synthese der Schutzgruppenreagenzien

### Allgemeine Arbeitsvorschrift (AAV) 2 zur Synthese der Schutzgruppenreagenzien

AAV 2: Zu einer auf 0 °C gekühlten Lösung von 6 mmol Chlorameisensäure-trichlormethylester (1,2-facher Überschuss) in 8 ml THF (dest. über CaH₂) wird innerhalb von 5 min eine Lösung von 5 mmol Alkohol und 5 mmol Triethylamin in 8 ml THF zugetropft. Nach 1 bis 2 stündigem Rühren im Eisbad (DC-Kontrolle) wird die Reaktionslösung über Kieselgur abgesaugt, mit etwas THF nachgewaschen und das überschüssige Reagens abrotiert. Die erhaltenen Schutzgruppenreagenzien werden 1 bis 2 h an der HV getrocknet und unter Argon im Eisschrank aufbewahrt.

### Beispiel C.1

### 2-(5-Chlor-2-nitrophenyl)propoxycarbonylchlorid (12)

Nach AAV 2: 914 mg (560-µl, 4,6 mmol)
Chlorameisensäuretrichlormethylester in 4 ml abs. THF, 664 mg (3,1 mmol) 2-(5-Chlor-2-nitrophenyl)propanol (9) und 311 mg (425 µl, 3,1 mmol) Triethylamin in 4 ml abs. THF.
Ausbeute: 838 mg (3 mmol, 97 %) (12) als gelbes Öl.

Physikalische Daten von (12):
DC (Kieselgel, CH₂Cl₂): R_{f} = 0,93
¹H-NMR (250 MHz, CDCl₃): 7,81 (d, 1H, H(3)), 7,39 (m, 2H, H(6), H(4)), 4,45 (m, 2H, α- CH₂), 3,84 (sextett, 1H, β-CH), 1,39 (d, 3H, CH₃)

### Beispiel C.2

### 2-(5-Brom-2-nitrophenyl)propoxycarbonylchlorid (13)

Nach AAV 2: 742 mg (455 µl, 3,8 mmol)
Chlorameisensäuretrichlormethylester in 4 ml abs. THF, 653 mg (2,5 mmol) 2-(5-Brom-2-nitrophenyl)propanol (10) und 254 mg (350 µl, 2,5 mmol) Triethylamin in 4 ml abs. THF, Ausbeute: 805 mg (2,49 mmol, 99 %) (13) als gelbes Öl.

Physikalische Daten von (13):
DC (Kieselgel, CH₂Cl₂): R_{f} = 0,92
¹H-NMR (250 MHz, CDCl₃): 7,72 (d, 1H, H(3)), 7,55 (m, 2H, H(6), H(4)), 4,45 (m, 2H, α-CH₂), 3,80 (sextett, 1H, β-CH), 1,39 (d, 3H, CH₃)

### Beispiel C.3

### 2-(5-Iod-2-nitrophenyl)propoxycarbonylchlorid (14)

Nach AAV 2: 760 mg (465 µl, 3,8 mmol)
Chlorameisensäuretrichlormethylester in 4 ml abs. THF, 787 mg (2,6 mmol) 2-(5-Iod-2-nitrophenyl)propanol (11) und 259 mg (355 µl, 2,6 mmol) Triethylamin in 4 ml abs. THF.
Ausbeute: 666 mg (1,8 mmol, 95 %) (14) als gelbes Öl.

Physikalische Daten von (14):
DC (Kieselgel, CH₂Cl₂) : R_{f} = 0,91
¹H-NMR (250 MHz, CDCl₃): 7,75 (m, 2H, H(6), H(4)),7,53 (d, 1H, H(3)), 4,44 (m, 2H, α-CH₂), 3,74 (sextett, 1H, β-CH), 1,38 (d, 3H, CH₃)

### Beispiel C.4

### 2-(2,5-Dinitrophenyl)propoxycarbonylchlorid (36)

Nach AAV 2: 237 mg (145 µl, 1,2 mmol)
Chlorameisensäuretrichlormethylester in 4 ml abs. THF, 226 mg (1 mmol) 2-(2,5-Dinitrophenyl)propanol (35) und 101 mg (140 µl, 1 mmol) Triethylamin in 4 ml abs. THF.
Ausbeute: 320 mg (1,1 mmol, 110 %) leicht verunreinigtes (36) als gelbes Öl.

Physikalische Daten von (36):
DC (Kieselgel, CH₂Cl₂): R_{f} = 0,98
¹H-NMR (250 MHz, CDCl₃): 8,34 (d, 1H, H(6)), 8,27 (dd, 1H, H(4)), 7,93 (d, 1H, H(3)), 4,43 (m, 2H, 2 x α-CH), 3,76 (sextett, 1H, β-CH ), 1,46 (d, 3H, CH₃)

### D) Synthese der 5'-O-geschützten-2'-Desoxynucleoside

### Allgemeine Arbeitsvorschriften (AAV) 3 zur Einführung der Schutzgruppenreagenzien in die 5'-O-Position der 2'-Desoxynucleoside

AAV 3: 1mmol Nucleosid wird 3 x mit je 3 ml abs. Pyridin koevaporiert, in 3 ml abs. Pyridin gelöst und auf -50 °C gekühlt. Dazu tropft man innerhalb von 15 min einen 1,25- bis 1,75-fachen Überschuss an Schutzgruppenreagens in 3 ml abs. CH₂Cl₂. Nach 5,5 h Rühren bei einer Temperatur zwischen -60°C und -30 °C, am Ende -15 °C, wird die Reaktionslösung mit 10 ml H₂O verdünnt und die wässrige Phase 3 x mit je 10 ml CH₂Cl₂ extrahiert. Die vereinigten organischen Phasen werden über Na₂SO₄ getrocknet, einrotiert und 3 x mit Toluol koevaporiert, um das Pyridin vollständig zu entfernen. Das erhaltene Rohprodukt wird über eine Kieselgelsäule mittels Flash-Chromatographie (20 g Kieselgel, 2 x 14 cm, LM: CH₂Cl₂/MeOH, kond.: CH₂Cl₂, Gradient: 100 ml CH₂Cl₂, je 100 ml 100:1, 100:2, 100:3, 100:3,5 und 100:4) gereinigt. Die entsprechenden Produktfraktionen werden einrotiert und die erhaltenen Schäume im HV bei 35 °C getrocknet.

### Beispiel D.1

### 5'-O-[2-(5-Chlor-2-nitrophenyl)propoxycarbonyl]-thymidin (15),

### Bis-[2-(5-chlor-2-nitrophenyl)propyl]-carbonat (16), 3',5'-Di-O-[2-(5-chlor-2-nitrophenyl)propoxycarbonyl]-thymidin (17), und 3'-O-[2-(5-Chlor-2-nitrophenyl)propoxycarbonyl]-thymidin (18)

Nach AAV 3: 970 mg (4 mmol) Thymidin/15 ml abs. Pyridin, 1,5 g (5 mmol) 2-(5-Chlor-2-nitrophenyl)propoxycarbonylchlorid (12)/15 ml abs. CH₂Cl₂.
Ausbeute:359 mg (0,8 mmol, 30 %) (16) als gelben Feststoff, 173 mg (0, 24 mmol, 6 %) (17), 70 mg (0,14 mmol, 4 %) (18) und 1,34 (2,8 mmol, 69 %) (15) als farblose Schäume.

Physikalische Daten von (15) :
DC (Kieselgel, Tol/EE/MeOH 5:4:1): R_{f} = 0,40
¹H-NMR (250 MHz, CDCl₃) : 8,75 (s (br.), 1H, NH), 7,74 (d, 1H, arom. H NPPOC), 7,41 (d, 1H, arom. H NPPOC), 7,34 (dd, 1H, arom. H NPPOC), 7,29 (s, 1H, H-C(6)), 6,31 (m, 1H, H-C(1')), 4,47-4,08 (m, 6H, α-CH₂ NPPOC, H-C(3'), 2 x H-C(5'), H-C(4')), 3,82 (m, 1H, β-CH NPPOC), 2,66 (d(br), 1H, OH-C(3')), 2,37 (m, 1H, H-C(2')), 2,17 (m, 1H, H-C(2')), 1,80 (dd, 3H, CH₃ Thy), 1,34 (d, 3H, CH₃ NPPOC)
UV-Spektrum (MeOH), λ ₘₐₓ[nm] (log ε) : 207 (4,32), 263 (4,15), [335 (2,92)]
C₂₀H₂₂ClN₃O₉ (483,86 g/mol) :
ber.: C 49,65, H 4,58, N 8,68; gef.: C 49,31, H 4,57, N 8,54

Physikalische Daten von (16) :
Schmp.: 95-100 °C
DC (Kieselgel, Tol/EE/MeOH 5:4:1) : R_{f} = 0,94
¹H-NMR (250 MHz, CDCl₃) : 7,75 (d, 2H, 2 x H(3)), 7,40 (m, 2H,2 x H(6)), 7,33 (m, 2H, 2 x H(4)), 4,32-4,15 (m, 4H, 2 x α-CH₂), 3,75 (m, 2H, 2 x β-CH), 1,33 (2 x d, 6H, 2 x CH₃) UV-Spektrum (MeOH), λ ₘₐₓ [nm] (log ε) : 203 (4,42), [215 (4,26)], 260 (4,07), [334 (3,23)]
C₁₉H₁₈Cl₂N₂O₇ (457,27 g/mol) :
ber.: C 49,91, H 3,97, N 6,13; gef.: C 49,93, H 3,99, N 6,11

Physikalische Daten von (17):
DC (Kieselgel, Tol/EE/MeOH 5:4:1): R_{f} = 0,79
¹H-NMR (250 MHz, CDCl₃): 8,03(s (br.), 1H, NH), 7,76 (m, 2H, 2 x arom. H NPPOC), 7,37 (m, 4H, 4 x arom. H NPPOC), 7,26 (s, 1H, H-C(6)), 6,32 (m, 1H, H-C(1')), 5,11 (m, 1H, H-C(3')), 4,46-4,12 (m, 7H, 2 x α-CH₂ NPPOC, 2 x H-C(5')), H-C(4')), 3,82 (m, 2H, 2 x β-CH NPPOC), 2,46 (m, 1H, H-C(2')), 2,22 (m, 1H, H-C(2')), 1,80 (d, 3H, CH₃ Thy), 1,36 (d, 6H, 2 x CH₃ NPOOC)
UV-Spektrum (MeOH), λ ₘₐₓ [nm] (log ε): 207 (4,64), 262 (4,32), [327 (3,34)]
C₃₀H₃₀Cl₂N₄O₁₃ (725,49 g/mol):
ber.: C 49,67, H 4,17, N 7,72; gef.: C 49,55, H 4,32, N 7,42

Physikalische Daten von (18):
DC (Kieselgel, Tol/EE/MeOH 5:4:1): R_{f} = 0,55
¹H-NMR (250 MHz, CDCl₃): 8,31(s (br.), 1H, NH), 7,78 (dd, 1H, arom. H NPPOC), 7,40 (m, 3H, 2 x arom. H NPPOC, H-C(6)), 6,12 (m, 1H, H-C(1')), 5,23 (m, 1H, H-C(3')), 4,38 (m, 1H, H-C(4')), 4,24-4,11 (m, 2H, α-CH₂ NPPOC), 3,84 (m, 3H, 2 x H-C(5'), β-CH NPPOC), 2,59-2,36 (m, 3H, OH-C(5'), 2 x H-C(2')), 1,90 (s, 3H, CH₃ Thy), 1,36(d, 3H, CH₃ NPOOC)
UV-Spektrum (MeOH), λ ₘₐₓ [nm] (log ε): 207 (4,37), 263 (4,17), [331 (2,96)]
C₂₀H₂₂ClN₃O₉ (483,86 g/mol):
ber.: C 49,65, H 4,58, N 8,68; gef.: C 49,58, H 4,70, N 8,02

### Beispiel D.2

### 5'-O-[2-(5-Brom-2-nitrophenyl)propoxycarbonyl]-thymidin (19),

### Bis-[2-(5-brom-2-nitrophenyl)propyl]-carbonat (20), 3',5'-Di-O-[2-(5-brom-2-nitrophenyl)propoxycarbonyl]-thymidin (21), und 3'-O-[2-(5-Brom-2-nitrophenyl)propoxycarbonyl]-thymidin (22)

Nach AAV 3: 1,18 g (4,9 mmol) Thymidin/20 ml abs. Pyridin, 2,1 g (6,6 mmol) 2-(5-Brom-2-nitrophenyl)propoxycarbonylchlorid (13)/20 ml abs. CH₂Cl₂. Ausbeute: 280 mg (0,5 mmol, 16 %) (20) als hellgelben Feststoff, 458 mg (0,56 mmol, 12 %) (21), 48 mg (0,1 mmol, 2 %) (22) und 1,89 g (3,6 mmol, 74 %) (19) als farblose Schäume.

Physikalische Daten von (19):
DC (Kieselgel, Tol/EE/MeOH 5:4:1): R_{f} = 0,42
¹H-NMR (250 MHz, CDCl₃) : 8,71 (s (br.), 1H, NH), 7,65 (d, 1H, arom. H NPPOC), 7,57 (d, 1H, arom. H NPPOC), 7,51 (dd, 1H, arom. H NPPOC), 7,29 (s, 1H, H-C(6)), 6,31 (m, 1H, H-C(1')), 5,28-4,11 (m, 6H, α-CH₂ NPPOC, H-C(3'), 2 x H-C(5'), H-C(4')), 3,80 (m, 1H, β-CH NPPOC), 2,64 (s(br), 1H, OH-C(3')), 2,37 (m, 1H, H-C(2')), 2,05 (m, 1H, H-C(2')), 1,80 (d, 3H, CH₃ Thy), 1,35 (d, 3H, CH₃ NPPOC)
UV-Spektrum (MeOH), λ max [nm] (log ε): 205 (4,41), 265 (4,18), [337 (2,95)]
C₂₀H₂₂BrN₃O₉ (528,32 g/mol):
ber.: C 45,47, H 4,20, N 7,95; gef.: C 45,09, H 4,14, N 7,54

Physikalische Daten von (20):
Schmp.: 92-97 °C
DC (Kieselgel, Tol/EE/MeOH 5:4:1): R_{f} = 0,93
¹H-NMR (250 MHz, CDCl₃): 7,66 (d, 2H, 2 x H(3)), 7,56 (d, 2H, 2 x H(6)), 7,48 (m, 2H, 2 x H(4)), 4,32-4,15 (m, 4H, 2 x α-CH₂), 3,71 (sextett, 2H, 2 x β-CH), 1,33 (2 x d, 6H, 2 x CH₃) UV-Spektrum (MeOH), λ ₘₐₓ [nm] (log ε) : 202 (4,49), [218 (4,26)], 263 (4,11), [334 (3,28)]
C₁₉H₁₈Br₂N₂O₇ (546,18 g/mol) :
ber.: C 41,78, H 3,32, N 5,13; gef.: C 42,01, H 3,35, N 4,96

Physikalische Daten von (21) :
DC (Kieselgel, Tol/EE/MeOH 5:4:1) : R_{f} = 0,78
¹H-NMR (250 MHz, CDCl₃) : 8,12 (s (br.), 1H, NH), 7,67 (m, 2H, 2 x arom. H NPPOC), 7,53 (m, 4H, 4 x arom. H NPPOC), 7,26 (m, 1H, H-C(6)), 6,33 (m, 1H, H-C(1')), 5,11 (m, 1H, H-C(3')), 4,46-4,11 (m, 7H, 2 x α-CH₂ NPPOC, 2 x H-C(5'), H-C(4')), 3,79 (m, 2H, 2 x β-CH NPPOC), 2,46 (m, 1H, H-C(2')), 2,22 (m, 1H, H-C(2')), 1,80 (d, 3H, CH₃ Thy), 1,35 (d, 6H, 2 x CH₃ NPPOC)
UV-Spektrum (MeOH), λ ₘₐₓ [nm] (log ε) : 202 (4,61), [216 (4,42)], 263 (4,34), [333 (3,33)]
C₃₀H₃₀Br₂N₄O₁₃ (814,40 g/mol) :
ber.: C 44,24, H 3,71, N 6,88; gef.: C 44,28, H 3,72, N 6,80

Physikalische Daten von (22) :
DC (Kieselgel, Tol/EE/MeOH 5:4:1) : R_{f} = 0,54
¹H-NMR (250 MHz, CDCl₃) : 7,98 (s (br.), 1H, NH), 7,69 (dd, 1H, arom. H NPPOC), 7,58 (d, 1H, arom. H NPPOC), 7,52 (dd, 1H, arom. H NPPOC), 7,38 (dd, 1H, H-C(6)), 6,12 (m, 1H, H-C(1')), 5,23 (m, 1H, H-C(3')), 4,38 (m, 1H-C(4')), 4,24-4,10 (m, 2H, α-CH₂ NPPOC) 3,91-3,74 (m, 3H, 2 x H-C(5'), β-CH NPPOC), 2,56-2,34 (m, 2H, 2 x H-C(2')), OH-C(5') nicht sichtbar, 1,91 (s, 3H, CH₃ Thy), 1,36 (d, 3H, CH₃ NPPOC) UV-Spektrum (MeOH), λ ₘₐₓ [nm] (log ε) : 202 (4,36), 264 (4,18), [333 (3,02)]
C₂₀H₂₂BrN₃O₉ (528,32 g/mol) :
ber.: C 45,47, H 4,20, N 7,95; gef.: C 45,15, H 4,21, N 7,57

### Beispiel D.3

### 5'-O-[2-(5-Iod-2-nitrophenyl)propoxycarbonyl]-thymidin (23),

### Bis-[2-(5-iod-2-nitrophenyl)propyl)-carbonat (24), 3',5'-Di-O-[2-(5-iod-2-nitrophenyl)propoxycarbonyl]-thymidin (25), und 3'-O-[2-(5-Iod-2-nitrophenyl)propoxycarbonyl]-thymidin (26)

Nach AAV 3: 1,12 g (4,6 mmol) Thymidin/20 ml abs. Pyridin, 2,3 g (6,2 mmol) 2-(5-Iod-2-nitrophenyl)propoxycarbonylchlorid (14)/20 ml abs. CH₂Cl₂. Ausbeute: 502 mg (0,8 mmol, 25 %) (24) als fast farblosen Feststoff, 458 mg (0,5 mmol, 11 %) (25), 75 mg (0,13 mmol, 3 %) (26) und 1,93 g (3,4 mmol, 73 %) (23) als farblose Schäume.

Physikalische Daten von (23) :
DC (Kieselgel, Tol/EE/MeOH 5:4:1): R_{f} = 0,46
¹H-NMR (250 MHz, CDCl₃) : 8,51 (s (br.), 1H, NH), 7,74 (m, 2H, 2 x arom. H NPPOC), 7,47 (d, 1H, arom. H NPPOC), 7,29 (d, 1H, H-C(6)), 6,31 (t, 1H, H-C(1')), 4,46-4,10 (m, 6H, α-CH₂ NPPOC, H-C(3'), 2 x H-C(5'), H-C(4')), 3,75 (m, 1H, β-CH NPPOC), 2,49 (s(br), 1H, OH-C(3')), 2,37 (m, 1H, H-C(2')), 2,18 (m, 1H, H-C(2')), 1,80 (m, 3H, CH₃ Thy), 1,35 (d, 3H, CH₃ NPPOC)
UV-Spektrum (MeOH), λ ₘₐₓ [nm] (log ε) : 204 (4,41), 267 (4,16), [335 (3,19)]
C₂₀H₂₂IN₃O₉ (575,31 g/mol) :
ber.: C 41,76, H 3,85, N 7,30; gef. : C 41,46, H 3,88, N 7,18

Physikalische Daten von (24) :
Schmp.: 104-115 °C
DC (Kieselgel, Tol/EE/MeOH 5:4:1): R_{f} = 0,94
¹H-NMR (250 MHz, CDCl₃): 7,76 (s, 2H, 2 x H(6)), 7,71 (d, 2H, 2 x H(4)), 7,49 (d, 2H, 2 x H(3)), 4,29 (m, 2H, α-CH₂ ), 4,17 (m, 2H, α-CH₂ ), 3,68 (sextett, 2H, 2 x β-CH), 1,34 (d, 6H, 2 x CH₃)
UV-Spektrum (MeOH), λₘₐₓ [nm] (log ε): 203 (4,57), [219 (4,26)], 276 (4,07), [332 (3,47)]
C₁₉H₁₈I₂N₂O₇ (640,16 g/mol):
ber.: C 35,65, H 2,83, N 4,38; gef.: C 36,04, H 3,06, N 4,07

Physikalische Daten von (25):
DC (Kieselgel, Tol/EE/MeOH 5:4:1): R_{f} = 0,84
¹H-NMR (250 MHz, CDCl₃): 8,26 (s (br.), 1H, NH), 7,74 (m, 4H, 4 x arom. H NPPOC), 7,49 (m, 2H, 2 x arom. H NPPOC), 7,27 (m, 1H, H-C(6)), 6,34 (m, 1H, H-C(1')), 5,12 (m, 1H, H-C(3')), 4,45-4,10 (m, 7H, 2 x α-CH₂ NPPOC, 2 x H-C(5'), H-C(4')), 3,75 (m, 2H, 2 x β-CH NPPOC), 2,45 (m, 1H, H-C(2')), 2,24 (m, 1H, H-C(2')), 1,80 (d, 3H, CH₃ Thy), 1,35 (d, 6H, 2 x CH₃ NPPOC)
UV-Spektrum (MeOH), λ ₘₐₓ [nm] (log ε): 202 (4,69), 267 (4,30), [333 (3,45)]
C₃₀H₃₀I₂N₄O₁₃ (908,38 g/mol) :
ber.: C 39,67, H 3,33, N 6,17; gef.: C 39,61, H 3,39, N 5,99

Physikalische-Daten von (26):
DC (Kieselgel, Tol/EE/MeOH 5:4:1): R_{f} = 0,53
¹H-NMR (250 MHz, CDCl₃): 7,78 (s (br.), 1H, NH), 7,73 (m, 2H, 2 x arom. H NPPOC), 7,51 (dd, 1H, arom. H NPPOC), 7,39 (dd, 1H, H-C(6)), 6,12 (m, 1H, H-C(1')), 5,23 (m, 1H, H-C(3')), 4,36 (m, 1H, α-CH NPPOC), 4,20 (m, 1H, α-CH NPPOC), 4,13 (m, 1H, H-C(4')), 3,87 (m, 2H, 2 x H-C(5')), 3,74 (sextett, 1H, β-CH NPPOC) , 2,56-2,39 (m, 3H, OH-C(5'), 2 x H-C(2')), 1,91 (s, 3H, CH₃ Thy), 1,35 (d, 3H, CH₃ NPPOC)
UV-Spektrum (MeOH), λₘₐₓ [nm] (log ε): 202 (4,49), 267 (4,17), [333 (3,19)]
C₂₀H₂₂IN₃O₉ (575,31 g/mol):
ber.: C 41,76, H 3,85, N 7,30; gef.: C 42,22, H 3,85, N 7,20

### Beispiel D.4

### 5'-O-[2-(2,5-Dinitrophenyl)propoxycarbonyl]-thymidin (37) und Bis[2-(2,5-dinitrophenyl)propyl)carbonat (38)

Nach AAV 3: 180 mg (0,74 mmol) Thymidin/3 ml abs. Pyridin, 320 mg (1 mmol) 2-(2,5-Dinitrophenyl)propoxycarbonylchlorid (36)/3 ml abs. CH₂Cl₂.
Ausbeute: 48 mg (0,1 mmol, 10 %) (38) als gelben Feststoff und 250 mg (0,51 mmol, 68 %) (37) als farblosen Schaum.

Physikalische Daten von (37) :
DC (Kieselgel, Tol/EE/MeOH 5:4:1) : R_{f} = 0,34
¹H-NMR (250 MHz, CDCl₃) : 9,11 (s(br), 1H, NH), 8,33 (d, 1H, H(6) NPPOC), 8,23 (m, 1H, H(4) NPPOC), 7,87 (dd, 1H, H(3) NPPOC) , 7,27 (s, 1H, H-C(6)), 6,28 (t, 1H, H-C(1')), 4,51-4,17 (m, 5H, 2 x α-CH NPPOC, H-C(3'), 2 x H-C(5')), 4,10
(m, 1H, H-C(4')), 3,74 (m, 1H, β-CH NPPOC), 3,03 (m(br), 1H, OH-C(3')), 2,38 (m, 1H, H-C(2')), 2,17 (m, 1H, H-C(2')), 1,79 (d, 3H, CH₃ Thy), 1,43 (d, 3H, CH₃ NPPOC)
UV-Spektrum (MeOH), λ ₘₐₓ [nm] (log ε): 205 (4,47), 258 (4,26), [334 (3,02)]
C₂₀H₂₂N₄O₁₁ x 0,5 H₂O (503,42 g/mol) :
ber.: C 47,71, H 4,60, N 11,13; gef.: C 48,08, H 4,67, N 11,01

### Beispiel D.5

### 5'-O-[2-(5-Chlor-2-nitrophenyl)propoxycarbonyl)-N⁶phenyloxyacetyl-2'-desoxyadenosin (27)

Nach AAV 3: 385 mg (1 mmol) N⁶-Phenyloxyacetyl-2'desoxyadenosin/3,5 ml abs. Pyridin, 403 mg (1,45 mmol) 2-(5-Chlor-2-nitrophenyl)propoxycarbonyl-chlorid (12)/3,5 ml abs. CH₂Cl₂.
Ausbeute: 463 mg (0,74 mmol, 74 %) (27) als farblosen Schaum.

Physikalische Daten von (27):
DC (Kieselgel, Tol/EE/MeOH 5:4:1): R_{f} = 0,46
¹H-NMR (250 MHz, CDCl₃) : 9,40 (s (br.), 1H, NH), 8,76 u. 8,16 (2 x d, 2H, H-C(2), H-C(8)), 7,74 (d, 1H, arom. H NPPOC), 7,41 (m, 1H, arom. H NPPOC), 7,32 (m, 3H, 1 x arom. H NPPOC, 2 x arom. H Pac), 7,04 (m, 3H, 3 x arom. H Pac), 6,49 (m, 1H, H-C(1')), 4,85 (s, 2H, CH₂ Pac) , 4,72 (m, 1H, H-C(3')), 4,42-4,08 (m, 5H, α-CH₂ NPPOC, 2 x H-C(5'), H-C(4')), 3,84 (m,
1H, β-CH NPPOC), 2,88 (m, 1H, H-C(2')), 2,56 (m, 1H, H-C(2')), 2,39 (s(br), 1H, OH-C(3')), 1,35 (d, 3H, CH₃ NPPOC) UV-Spektrum (MeOH), λ ₘₐₓ [nm] (log ε) : 209 (4,61), [260 (4,30)], 270 (4,38), [337 (2,94)]
C₂₈H₂₇ClN₆O₉ (627,01 g/mol):
ber.: C 53,64, H 4,34, N 13,40; gef.: C 53,44, H 4,30, N 13,50

### Beispiel D.6

### 5'-O-[2-(5-Brom-2-nitrophenyl)propoxycarbonyl]-N⁶phenyloxyacetyl-2'-desoxyadenosin (28)

Nach AAV 3: 319 mg (0,83 mmol) N⁶-Phenyloxyacetyl-2'desoxyadenosin/3 ml abs. Pyridin, 374 mg (1,16 mmol) 2-(5-Brom-2-nitrophenyl)propoxycarbonyl-chlorid (13)/3 ml abs. CH₂Cl₂.
Ausbeute: 397 mg (0,59 mmol, 71 %) (28) als farblosen Schaum.

Physikalische Daten von (28):
DC (Kieselgel, Tol/EE/MeOH 5:4:1): R_{f} = 0,31
¹H-NMR (250 MHz, CDCl₃) : 9,44 (s (br.), 1H, NH), 8,75 u. 8,18 (2 x d, 1H, H-C(2), H-C(8)), 7,65 (d, 1H, arom. H NPPOC), 7,58 (dd, 1H, arom. H NPPOC), 7,47 (m, 1H, arom. H NPPOC), 7,31 (m, 2H, 2 x arom. H Pac), 7,02 (m, 3H, 3 x arom. H Pac), 6,50 (t, 1H, H-C(1')), 4,85 (s, 2H, CH₂ Pac) , 4,71 (m, 1H, H-C(3')), 4,44-4,08 (m, 5H, α-CH₂ NPPOC, 2 x H-C(5'), H-C(4')), 3,80 (m, 1H, β-CH NPPOC), 2,87 (m, 1H, H-C(2')), 2,57 (m, 1H, H-C(2'), OH-C(3') (verdeckt), 1,35 (d, 3H, CH₃ NPPOC) UV-Spektrum (MeOH), λₘₐₓ [nm] (log ε): 209 (4,62), [258 (4,29)], 270 (4,39), 335 (3,06)
C₂₈H₂₇BrN₆O₉ (671,46 g/mol):
ber.: C 50,09, H 4,05, N 12,52; gef.: C 49,86, H 4,04, N 12,57

### Beispiel D.7

### 5'-O-[2-(5-Iod-2-nitrophenyl)propoxycarbonyl]-N⁶phenyloxyacetyl-2'-desoxyadenosin (29)

Nach AAV 3: 310 mg (0,8 mmol) N⁶-Phenyloxyacetyl-2'desoxyadenosin/3 ml abs. Pyridin, 429 mg (1,16 mmol) 2-(5-Iod-2-nitrophenyl)propoxycarbonylchlorid (14)/3 ml abs CH₂Cl₂.
Ausbeute: 451 mg (0,63 mmol, 78 %) (29) als farblosen Schaum.

Physikalische Daten von (29) :
DC (Kieselgel, Tol/EE/MeOH 5:4:1) : R_{f} = 0,44
¹H-NMR (250 MHz, CDCl₃): 9,43 (s (br.), 1H, NH), 8,75 u. 8,18 (2 x d, 2H, H-C(2), H-C(8)), 7,76 (m, 1H, arom. H NPPOC), 7,69 (m, 1H, arom. H NPPOC), 7,47 (d, 1H, arom. H NPPOC), 7,32 (m, 2H, 2 x arom. H Pac), 7,02 (m, 3H, 3 x arom. H Pac), 6,51 (t, 1H, H-C(1')), 4,85 (s, 2H, CH₂ Pac), 4,72 (m, 1H, H-C(3')), 4,44-4,07 (m, 5H, α-CH₂ NPPOC, 2 x H-C(5'), H-C(4')), 3,75 (m, 1H, β-CH NPPOC), 2,87 (m, 1H, H-C(2')), 2,68 (s(br), 1H, OH-C(3')), 2,57 (m, 1H, H-C(2')), 1,34 (d, 3H, CH₃ NPPOC)
UV-Spektrum (MeOH), λ ₘₐₓ [nm] (log ε) : 209 (4,62), [258 (4,29)], 270 (4,39), 335 (3,06)
C₂₈H₂₇IN₆O₉ (718,46 g/mol) :
ber.: C 46,81, H 3,79, N 11,70; gef.: C 46,64, H 3,79, N 11,73

### Bestrahlungsexperimente

### 1. Durchführung

Die entsprechend geschützten Nucleosid-Derivate wurden mit Hilfe einer Apparatur bestrahlt, die aus einer Hg-Höchstdrucklampe (200 W), einem IR-Filter (Wasser), einem Shutter (automatischer Verschluss zur exakten Regelung der Bestrahlungsdauer), einem Standard-Interferenzfilter (Filter 1) mit einem schmalen Bereich um die Wellenlänge 365 nm, einer Sammellinse sowie einem auf ca. 17 °C thermostatisierten Küvettenhalter bestand. Um die Überhitzung von Filter 1 zu verhindern, wurde ggf. zwischen Shutter und Filter 1 noch ein Breitbandfilter UG1 (Filter 2) installiert. Bei den Bestrahlungsversuchen wurde Licht der Wellenlänge 365 nm verwendet, damit nur die Schutzgruppe und nicht die heterocyclischen Basen angeregt werden. Die Bestrahlung erfolgte in Quarzküvetten (3,5 ml) mit je 3 ml Lösung (Lösemittel: Methanol/Wasser 1 : 1; Ausgangskonzentration 0,1 mmol/l Nach erfolgter Bestrahlung wurden der Küvette zwei Proben entnommen und mit Hilfe eines HPLC-Systems analysiert.

Das HPLC-System der Fa. Merck-Hitachi bestand aus folgenden Geräten:
Pumpe L-7100, Auto-Sampler L-7200, UV/VIS-Detektor (Detektionswellenlänge 260 nm) L-7420 und Interface D-7000.
Als Säule wurde eine LICHROSORB RP 18 der Fa. Merck verwendet. Die Steuerung erfolgte mit einem Computer der Fa. Compaq durch den HSM-Manager.

Zur Chromatographie wurde der folgende Gradient (Lösemittel: Wasser und Acetonitril) verwendet (s. Tabelle 1).

**Tabelle 1**

| **Gradient** | | | | |
|---|---|---|---|---|
| **Zeit** **[min]** | **H**_{**2**}**O** | **H**_{**2**}**O/MeCN (1:1) [%]** | **MeCN [%]** | **Fluss** |
| 0 | 100 | 0 | 0 | 1 |
| 3 | 100 | 0 | 0 | 1 |
| 10 | 0 | 100 | 0 | 1 |
| 25 | 0 | 0 | 100 | 1 |
| 30 | 0 | 100 | 0 | 1 |
| 33 | 100 | 0 | 0 | 1 |
| 38 | 100 | 0 | 0 | 1 |

In den erhaltenen Chromatogrammen konnten die Abnahme des Eduktes (5'-O-geschütztes Nucleosid) und die Zunahme des Produktes (5'-O-entschütztes Nucleosid) verfolgt werden. Die Auswertung erfolgt dabei über die Fläche der einzelnen Peaks. Als Referenz wurde die Lösung des zu bestrahlenden Nucleosids zum Zeitpunkt 0 Min. (d. h. vor der Bestrahlung) eingespritzt und die Fläche des erhaltenen Peaks als 100 % Edukt angesehen. Gleichermaßen wurde für das Produkt verfahren: Es wurde die Peakfläche einer 0,1 mmolaren Lösung bestimmt und als 100 % gesetzt. Die jeweiligen Flächen der Produkte und Edukte der anderen Zeitpunkte wurden auf diese Referenzwerte bezogen.

Aus den so erhaltenen Kurven (Konz. in % gegen die Zeit aufgetragen) wurden folgende Werte abgelesen:
t_{H}: Halbwertszeit: der Zeitpunkt, an dem die Hälfte des Eduktes umgesetzt war.
Konz. t_{end}: Konzentration des Produktes am letzten untersuchten Zeitpunkt.
Meistens wurde dieser Zeitpunkt so gelegt, dass das Edukt nicht mehr nachweisbar war.

Die Ergebnisse der Bestrahlungsexperimente sind in Tabelle 2 zusammengefasst.

Wie man aus Tabelle 2 ersieht, variieren die Halbwertszeiten bei den verschiedenen Nucleosiden nur gering. Während das 5'-O-[2-(5-Iod-2-nitrophenyl)propoxycarbonyl]-Derivat (Verbindung 29) mit 23 Sekunden die kürzeste Halbwertszeit aufweist, beträgt diese beim 5'-O-[2-(2,5-Dinitrophenyl)propoxycarbonyl]thymidin (Verbindung 37) 44 Sekunden.

Was die Ausbeuten der entschützten Nucleoside betrifft, so lässt Tabelle 2 erkennen, dass beim 5'-O-[2-(5-Chlor-2-nitrophenyl)propoxycarbonyl]-N⁶-phenyloxyacetyl-2'desoxyadenosin (Verbindung 27) diese mit 97 % am höchsten liegt, während sie bei den anderen Nucleosid-Derivaten Werte zwischen ca. 75 und 95 % annimmt.

**Tabelle 2:**

| Ergebnisse der Bestrahlungsexperimente: | | | |
|---|---|---|---|
| Beispiel | Verbindung | Halbwertszeit t_{H} | Konz. t_{end} (t_{end}) |
| D1 | 5'-O-[2-(5-Chlor-2-nitrophenyl)propoxycarbonyl]thymidin (15) | 38 sec. | 91 % (5 min) |
| D2 | 5'-O-[2-(5-Brom-2-nitrophenyl)propoxycarbonyl]thymidin (19) | 32 sec. | 94 % (5 min) |
| D3 | 5'-O-[2-(5-Iod-2-nitrophenyl)propoxycarbonyl]thymidin (23) | 25 sec. | 86 % (5 min) |
| D4 | 5'-O-[2-(2,5-Dinitrophenyl)propoxycarbonyl]thymidin (37) | 44 sec. | 75 % (10 min) |
| D5 | 5'-O-[2-(5-Chlor-2-nitrophenyl)propoxycarbonyl]-N⁶-phenyloxyacetyl-2'-desoxyadenosin (27) | 35 sec. | 97 % (5 min) |
| D6 | 5'-O-[2-(5-Brom-2-nitrophenyl)propoxycarbonyl]-N⁶-phenyloxyacetyl-2'-desoxyadenosin (28) | 30 sec. | 95 % (5 min) |
| D7 | 5'-O-[2-(5-Iod-2-nitrophenyl)propoxycarbonyl]-N⁶-phenyloxyacetyl-2'-desoxyadenosin (29) | 23 sec. | 95 % (5 min) |

## Patentansprüche

1. Verbindung der allgemeinen Formel (I) 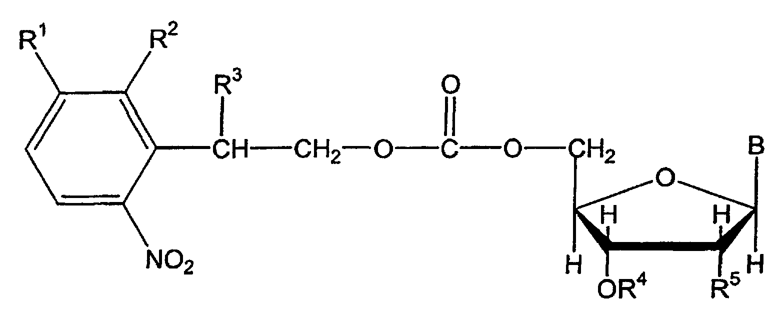 mit
R¹ = H, F, Cl, Br, I, NO₂
R² = H, CN
wobei R¹ und R² nicht gleichzeitig H sind
R³ = H, Alkylrest mit 1 bis 4 C-Atomen, Phenyl
R⁴ = H oder eine übliche funktionelle Gruppe zur Herstellung von Oligonucleotiden
R⁵ = H, OH, Halogen oder XR⁶, wobei X = O oder S und R⁶ eine in der Nucleotidchemie übliche Schutzgruppe darstellt,
B = Adenin, Cytosin, Guanin, Thymin, Uracil, 2,6-Diaminopurin-9-yl, Hypoxanthin-9-yl, 5-Methylcytosin-1-yl, 5-Amino-4-imidazolcarbonsäureamid-1-yl oder 5-Amino-4-imidazolcarbonsäureamid-3-yl, wobei im Falle von B = Adenin, Cytosin oder Guanin die primäre Aminofunktion ggf. eine permanente Schutzgruppe aufweist.

2. Verbindung nach Anspruch 1, wobei R⁴ eine Phosphitamidgruppe der Formel: 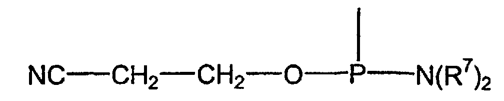 oder 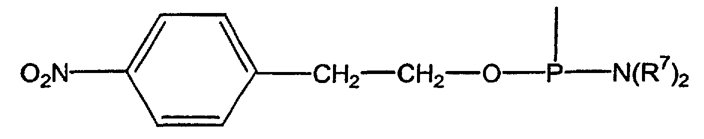 darstellt, wobei die R⁷-Gruppen gleich oder verschieden sind und lineare oder verzweigte Alkylreste mit 1 bis 4 C-Atomen bedeuten.

3. Verbindung nach den Ansprüchenl oder 2, wobei R⁵ eine Gruppe XR⁶ ist und R⁶ im Fall von X = O eine Alkyl-, Alkenyl-, Acetal- oder Silylether-Schutzgruppe oder im Fall von X = S eine Alkyl-Schutzgruppe darstellt.

4. Verbindung nach Anspruch 3, wobei als R⁵ ein O-Methyl- oder O-Ethylrest, ein O-Allylrest, ein O-Tetrahydropyranyl- bzw. O-Methoxytetrahydropyranyl-Rest oder ein O-t-Butyldimethylsilyl-Rest eingesetzt wird.

5. Verbindung nach den Ansprüchen 1 bis 4, wobei im Falle von B = Adenin, Cytosin oder Guanin als permanente Schutzgruppe Phenoxyacetyl-, 4-tert-Butyl-phenoxyacetyl-, 4-Isopropyl-phenoxyacetyl- oder Dimethylformamidino-Reste, im Falle von B = Adenin als permanente Schutzgruppe Benzoyl- oder p-Nitrophenylethoxycarbonyl-(p-NPEOC)-Reste, im Falle von B = Guanin als permanente Schutzgruppe Isobutyroyl-, p-Nitrophenylethyl (p-NPE) oder p-NPEOC-Reste und im Falle von B = Cytosin als permanente Schutzgruppe Benzoyl-, Isobutyroyl- oder p-NPEOC-Reste eingesetzt wird.

6. Verfahren zur Herstellung von Verbindungen nach den Ansprüchen 1 bis 5 in mindestens zwei Stufen, wobei man
a) einen Alkohol der allgemeinen Formel (II) 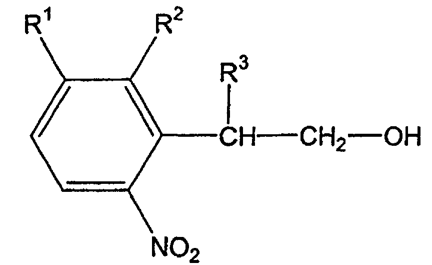 in der R¹, R², R³ die oben angegebene Bedeutung besitzen, mit einem Phosgen-Derivat umsetzt und anschließend
b) den in Stufe a) gebildeten Chlorkohlensäureester mit Nucleosiden der allgemeinen Formel (III) 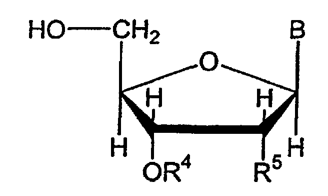 in der R⁴, R⁵ und B die oben angegebene Bedeutung besitzen, reagieren lässt und ggf. anschließend
c) in der 3'-Stellung der Nucleosid-Derivate mit R⁴ = H die Phosphitamid-Gruppe 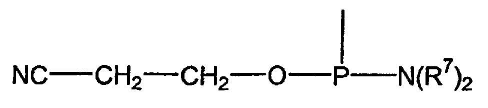 oder 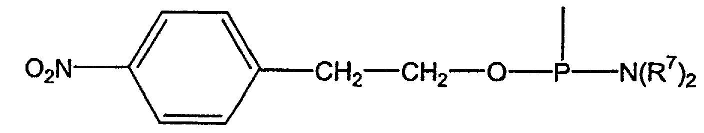
worin R⁷ die oben angegebene Bedeutung hat, nach bekannten Methoden einführt.

7. Verfahren nach Anspruch 6, wobei man Stufe a) in einem unpolaren organischen Lösemittel, wie beispielsweise Toluol oder THF, bei Temperaturen zwischen - 20 und + 25 °C durchführt und/oder das Phosgenderivat in zwei- bis fünffachem Überschuss bezogen auf die Alkoholkomponente einsetzt.

8. Verfahren nach den Ansprüchen 6 oder 7, wobei man Stufe b) in einem Lösemittelgemisch bestehend aus Dichlormethan und einem polaren organischen Lösemittel, wie beispielsweise DMF oder Pyridin, bei Temperaturen zwischen - 60 und + 25 °C durchführt.

9. Verfahren nach den Ansprüchen 6 bis 8, wobei man in Stufe b) ein Lösemittelgemisch bestehend aus Dichlormethan und DMF sowie eine Base ausgewählt aus der Gruppe Pyridin, Triethylamin und Ethyldiisopropylamin einsetzt.

10. Verfahren nach den Ansprüchen 6 bis 9, wobei das Mischungsverhältnis Dichlormethan zu Pyridin bzw. DMF 1 : 1 bis 3 : 1 und/oder das Molverhältnis Nucleosid zu Chlorkohlensäureester 1 : 1 bis 1 : 2 beträgt.

11. Verfahren nach den Ansprüchen 6 bis 10, wobei man in Stufe b) das in Pyridin oder DMF/Base gelöste Nucleosid vorlegt und eine Lösung des Chlorkohlensäureesters in Dichlormethan bei der jeweiligen Reaktionstemperatur zutropfen lässt.

12. Verfahren nach den Ansprüchen 6 bis 11, wobei man die Einführung der Phosphitamid-Gruppe (Stufe c)) durch Umsetzung der Nucleosid-Derivate (mit R⁴ = H) mit den entsprechenden Phosphinen in Gegenwart von 1H-Tetrazol als Aktivator in einem Lösemittelgemisch bestehend aus Dichlormethan und Acetonitril bei Temperaturen zwischen 0 und 25 °C vornimmt.

13. Verwendung einer Verbindung der allgemeinen Formel (I) gemäß Anspruch 1 für die lichtgesteuerte Synthese von Oligonucleotiden.

14. Verwendung gemäß Anspruch 13, wobei die lichtgesteuerte Oligonucleotidsynthese auf einem festen Trägermaterial erfolgt.

## Revendications

1. Composé de formule générale (I), 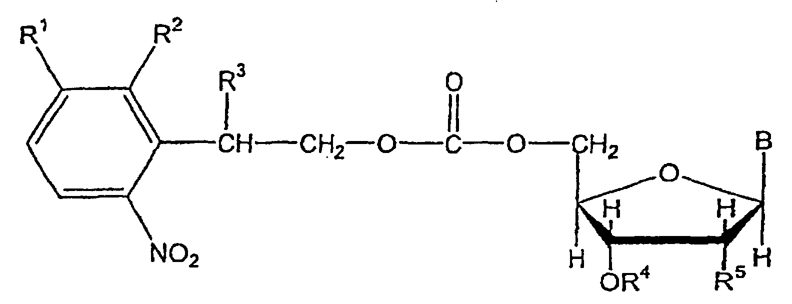 avec
R¹ = H, F, Cl, Br, I, NO₂
R² = H, CN,
sachant que R¹ et R² ne sont pas simultanément H,
R³ = H, un résidu alkyle ayant de 1 à 4 atomes C, un radical phényle
R⁴ = H ou un groupe fonctionnel usuel pour la préparation d'oligonucléotides
R⁵ = H, OH, un halogène ou XR⁶, sachant que X = 0 ou S et que R⁶ représente un groupe protecteur usuel dans la chimie des nucléotides,
B = adénine, cytosine, guanine, thymine, uracile, 2,6-diaminopurin-9-yl, hypoxanthin-9-yl, 5-méthyl-cytosin-1-yl, 5-amino-4-imidazol acide carboné amide-1-yl ou 5-amino-4-imidazol acide carboné amide-3-yl, sachant que, dans le cas où B = adénine, cytosine, ou guanine, la fonction amine primaire présente le cas échéant un groupe protecteur permanent.

2. Composé selon la revendication 1, R⁴ étant un groupe phosphitamide de formule : 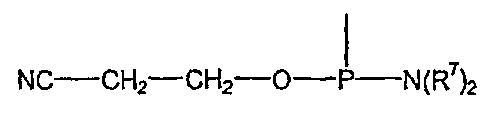 ou 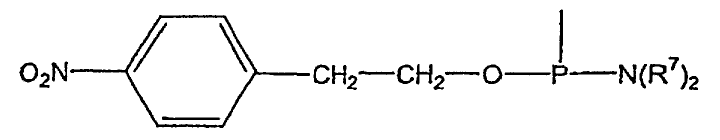 sachant que les groupes R⁷ sont identiques ou différents et signifient des résidus alkyle linéaires ou ramifiés comportant de 1 à 4 atomes C.

3. Composé selon la revendication 1 ou 2, R⁵ étant un groupe XR⁶ et R⁶, dans le cas de X = 0, étant un groupe protecteur alkyl, alkényl, acétal, ou silyléther, ou bien représentant un groupe protecteur alkyl dans le cas où X = S.

4. Composé selon la revendication 3, sachant qu'on utilise pour R⁵ un résidu O-méthyl ou O-éthyl, un résidu O-allyl, un résidu O-tétrahydro(xy*)pyranyl ou O-méthoxytétra-hydropyranyl ou un résidu O-t-butyldiméthylsilyl.

5. Composé selon les revendications 1 à 4, où, dans le cas de B = adénine, cytosine, ou guanine, on utilise en tant que groupe protecteur permanent des résidus phénoxyacétyl, 4-tert-butyl-phénoxyacétyl, 4-isopropylphénoxyacétyl ou diméthylformamidino, dans le cas de B = adénine, on utilise comme groupe protecteur permanent des résidus benzoyl ou p-nitrophényléthoxycarbonyl-(p-NPEOC), dans le cas ou B = guanine, on utilise comme groupe protecteur permanent des résidus isobutyroyl, p-nitrophényléthyl (p-NPE) ou p-NPEOC et, dans le cas où B = cytosine, on utilise comme groupe protecteur permanent des résidus benzoyl, isobutyroyl ou p-NPEOC.

6. Procédé de préparation de composé selon les revendications 1 à 5 en au moins deux étapes, où
a) un alcool de formule générale (II) 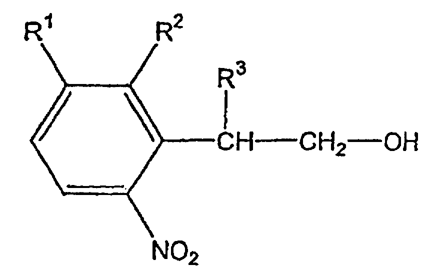 dans lequel R¹, R², R³ ont la signification indiquée ci-dessus est convertit avec un dérivé phosgène et, ensuite,
b) on laisse réagir l'ester d'acide carbonique chloré formé à l'étape a) avec des nucléosides de formule générale (III) 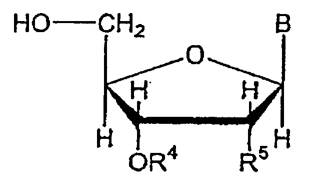 dans laquelle R⁴, R⁵ et B ont la signification indiquée ci-dessus et, le cas échéant, ensuite,
c) à la position 3' des dérivés nucléosides avec R⁴ = H, on introduit d'après des méthodes connues le groupe phosphitamide 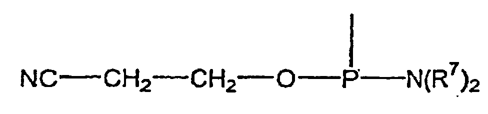
ou 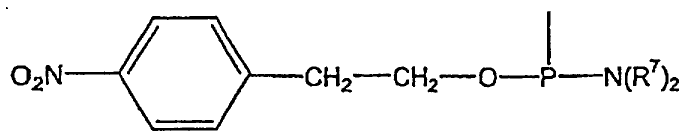 dans lequel R⁷ a la signification indiquée ci-dessus.

7. Procédé selon la revendication 6, où l'on exécute l'étape a) dans un solvant organique non polaire, tel que par exemple du toluène ou du THF, à des températures comprises entre - 20 et + 25 °C et/ou on introduit le dérivé phosgène en un excès allant du double au quintuple, si l'on se réfère au composant alcoolique.

8. Procédé selon la revendication 6 ou 7, où l'on exécute l'étape b) dans un solvant formé de dichlorométhane et d'un solvant organique polaire, tel que, par exemple, le DMF ou la pyridine, à des températures comprises entre - 60 et + 25 °C.

9. Procédé selon les revendications 6 à 8, où, à l'étape b), on utilise un mélange de solvant formé de dichlorométhane et de DMF, ainsi que d'une base sélectionnée dans le groupe de la pyridine, triéthylamine et éthyldiisopropylamine.

10. Procédé selon les revendications 6 à 9, le rapport de mélange entre le dichlorométhane et la pyridine ou le DMF étant de 1 : 1 à 3 : 1 et/ou le rapport molaire entre nucléoside et ester d'acide chlorocarboné étant de 1 : 1 à 1 : 2.

11. Procédé selon les revendications 6 à 10, où à l'étape b), on présente le nucléoside dissout dans la pyridine ou le DMF/base et on ajoute goutte à goutte une solution d'ester d'acide chlorocarboné dans le dichlorométhane, à la température de réaction respective.

12. Procédé selon les revendications 6 à 11, où l'on effectue l'introduction du groupe phosphitamide (étape c)) par conversion des dérivés nucléosides (avec R⁴ = H) avec les phosphines correspondantes, en présence de 1H-thétrazol en tant qu'activateur, dans un mélange de solvant formé de dichlorométhane et d'acétonitrile, à des températures comprises entre 0 et 25 °C.

13. Utilisation d'un composé de formule générale (I) selon la revendication 1 pour la synthèse photocommandée des oligonucléotides.

14. Utilisation selon la revendication 13, la synthèse photocommandée des oligonucléotides se faisant sur un matériau support solide.

## Claims

1. Compound of the general formula (I) 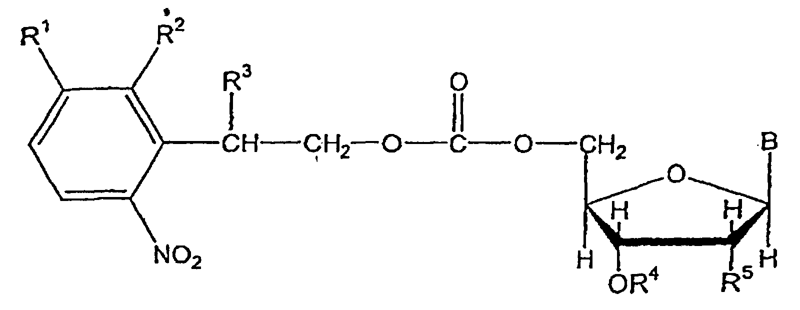 where
R¹ = H, F, Cl, Br, I, NO₂
R² = H, CN
wherein R¹ and R² are not H at the same time
R³ = H, alkyl radical having 1 to 4 C atoms, phenyl
R⁴ = H or a conventional functional group for the production of oligonucleotides
R⁵ = H, OH, halogen or XR⁶, wherein X = O or S and R⁶ represents a protective group conventional in nucleotide chemistry,
B = adenine, cytosine, guanine, thymine, uracil, 2,6-diaminopurin-9-yl, hypoxanthin-9-yl, 5-methylcytosin-1-yl, 5-amino-4-imidazolcarboxylic acid amid-1-yl or 5-amino-4-imidazolcarboxylic acid amid-3-yl, wherein in the case of B = adenine, cytosine or guanine, the primary amino function optionally has a permanent protective group.

2. Compound according to claim 1, wherein R⁴ represents a phosphite amide group of the formula: 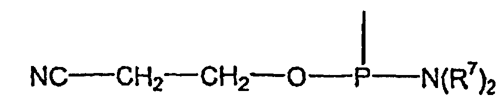 or 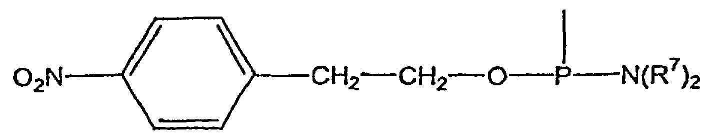 wherein the R⁷ groups are the same or different and denote linear or branched alkyl radicals having 1 to 4 C atoms.

3. Compound according to claims 1 or 2, wherein R⁵ is a group XR⁶ and R⁶ in the case of X=O represents an alkyl, alkenyl, acetal or silyl ether protective group or in the case of X = S represents an alkyl protective group.

4. Compound according to claim 3, wherein an O-methyl or O-ethyl radical, an O-allyl radical, an O-tetrahydropyranyl or O-methoxytetrahydropyranyl radical or an O-t-butyl dimethylsilyl radical is used as R⁵.

5. Compound according to claims 1 to 4, wherein in the case of B = adenine, cytosine or guanine, phenoxyacetyl, 4-tert-butyl-phenoxyacetyl, 4-isopropylphenoxyacetyl or dimethylformamidino radicals, are used as permanent protective group, in the case of B = adenine, benzoyl, or p-nitrophenylethoxycarbonyl-(p-NPEOC) radicals are used as permanent protective group, in the case of B = guanine, isobutyroyl, p-nitrophenylethyl (p-NPE) or p-NPEOC radicals are used as permanent protective group and in the case of B = cytosine, benzoyl, isobutyroyl or p-NPEOC radicals are used as permanent protective group.

6. Process for producing compounds according to claims 1 to 5 in at least two stages, wherein
a) an alcohol of the general formula (II) 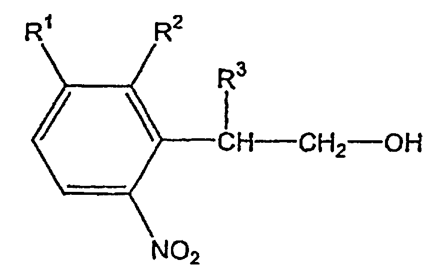 in which R¹, R², R³ have the meaning indicated above, is reacted with a phosgene derivative and then
b) the chlorocarbonate formed in stage a) is allowed to react with nucleosides of the general formula (III) 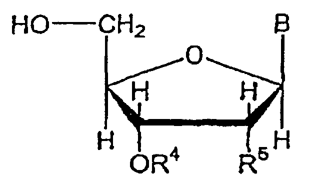 in which R⁴, R⁵ and B have the meaning indicated above, and optionally then
c) in the 3' position of the nucleoside derivatives where R⁴ = H, the phosphite amide group 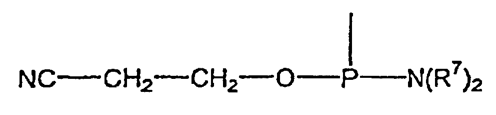 or 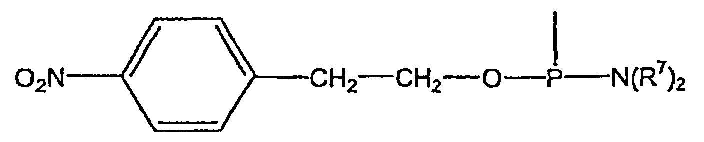
wherein R⁷ has the meaning indicated above, is introduced by known methods.

7. Process according to claim 6, wherein stage a) is carried out in a non-polar organic solvent, such as for example toluene or THE, at temperatures between -20 and +25°C and/or the phosgene derivative is used in two to five times excess based on the alcohol component.

8. Process according to claims 6 or 7, wherein stage b) is carried out in a solvent mixture consisting of dichloromethane and a polar organic solvent, such as for example DMF or pyridine, at temperatures between -50 and +25°C.

9. Process according to claims 6 to 8, wherein in stage b), a solvent mixture consisting of dichloromethane and DMF is used as well as a base selected from the group pyridine, triethylamine and ethyl diisopropylamine.

10. Process according to claims 6 to 9, wherein the mixing ratio of dichloromethane to pyridine or DMF is 1 : 1 to 3 : 1 and/or the molar ratio of nucleoside to chlorocarbonate is 1 : 1 to 1 : 2.

11. Process according to claims 6 to 10, wherein in stage b), the nucleoside dissolved in pyridine or DMF/base is initially provided and a solution of chlorocarbonate in dichloromethane is allowed to be added dropwise at the particular reaction temperature.

12. Process according to claims 6 to 11, wherein the introduction of the phosphite amide group (stage c)) is carried out by reacting the nucleoside derivatives (where R⁴ = H) with the corresponding phosphines in the presence of 1H-tetrazole as activator in a solvent mixture consisting of dichloromethane and acetonitrile at temperatures between 0 and 25°C.

13. Use of a compound of the general formula (I) according to claim 1 for the light-controlled synthesis of oligonucleotides.

14. Use according to claim 13, wherein the light-controlled oligonucleotide synthesis takes place on a solid carrier material.
